# EUROPEAN PATENT APPLICATION

(11) **EP 4 843 559 A2**
(43) Date of publication of application: **25.11.2026**
(21) Application number: 26211598.3
(22) Date of filing: 27.06.2024
(51) Int. Cl.: A61K 8/86

(54) **ORAL CARE COMPOSITIONS COMPRISING HOPS BETA ACIDS**

(30) Priority: 28.06.2023 US 202363510644 P
(62) Document number(s) of the earlier application(s) in accordance with Art. 76 EPC: 24748724.2 / 4 734 939
(71) Applicant: The Procter & Gamble Company, Cincinnati, OH 45202 (US)
(72) Inventor: BAIG, Arif Ali, Cincinnati, 45202 (US); STRAND, Ross, 138547 SINGAPORE (SG); STJOHN, Samuel James, Cincinnati, 45202 (US)
(74) Representative: P&G Patent Germany

(57) **Abstract**

An oral care composition includes hops and one or more surfactants. A solution including the hops and the one or more surfactants is clear or slightly hazy in a pH range of about 4.5 to about 9. If the one or more surfactants include sodium cocoyl glutamate, lauryl glucoside, or poloxamer, then the oral care composition includes at least two surfactants.

## Description

### TECHNICAL FIELD

The disclosure relates generally to oral care compositions and, more particularly, relates to oral care compositions comprising hops and surfactant with improved hops solubility.

### BACKGROUND

Oral care compositions, such as toothpaste and/or dentifrice compositions, can be applied to the oral cavity to clean and/or maintain the aesthetics and/or health of the teeth, gums, and/or tongue. Additionally, many oral care compositions are used to deliver active ingredients directly to oral care surfaces. Natural compounds with antibacterial activity, such as hops, can be incorporated into oral care compositions to provide antibacterial and/or anticavity activity. Natural antibacterial agents, such as hops, can include mixtures of active compounds, oils, flavonoids, and/or other flavor compounds. Incorporating natural compounds into oral care compositions may affect the shelf-stability of the compositions. There is a need for oral care compositions with improved shelf-stability.

### SUMMARY

An oral care composition is provided that includes hops and one or more surfactants, where a solution including the hops and the one or more surfactants is clear or slightly hazy in a pH range of about 4.5 to about 9.

An oral care composition is provided that includes hops and one or more surfactants including from greater than 0% to less than 2% of sodium methyl cocoyl taurate, where a solution comprising the hops and the one or more surfactants is clear or slightly hazy in a pH range of about 4.5 to about 9.

### DETAILED DESCRIPTION

Many natural antibacterial agents, including hops, have poor aqueous solubility and can phase separate from the toothpaste, collecting in the tip or partitioning along the composition/package interface. This results in a non-uniform distribution of the natural antibacterial agent such that the consumer cannot be guaranteed that they are receiving the proper amount of the agent during use. Embodiments of the present invention are directed to oral care compositions comprising one or more surfactants with hops beta acids, e.g., lupulone-sodium lauryl sulfate, adlupulone-cocamidopropyl betaine, or colupulone-sodium methyl cocoyl taurate, and/or others, that have improved hops stability. Solutions where hops beta acids are properly solubilized will improve the physical stability of the oral care composition and help prevent its phase separation in an aqueous composition.

While not wishing to being bound by theory, it is believed that the combination of one or more surfactants with a hops beta acid extract can lead to increased phase stability of oral care compositions comprising a hops beta acid extract.

The stability in a dentifrice composition may be assessed based on a liquid composition without colloidal stabilizers like abrasive and polymer binders. Such a composition may be considered sufficiently stable that there would not be bulk phase separation in the presence of colloidal stabilizers like abrasive and polymer binders where there is no visible separation, where discontinuous droplets are present, or a combination thereof. A composition that separates into continuous layers would be considered to lack phase stability.

### Definitions

To define more clearly the terms used herein, the following definitions are provided. Unless otherwise indicated, the following definitions are applicable to this disclosure. If a term is used in this disclosure but is not specifically defined herein, the definition from the IUPAC Compendium of Chemical Terminology, 2nd Ed (1997), can be applied, as long as that definition does not conflict with any other disclosure or definition applied herein, or render indefinite or non-enabled any claim to which that definition is applied.

The term "oral care composition", as used herein, includes a product, which in the ordinary course of usage, is not intentionally swallowed for purposes of systemic administration of particular therapeutic agents, but is rather retained in the oral cavity for a time sufficient to contact dental surfaces or oral tissues. Examples of oral care compositions include dentifrice, toothpaste, tooth gel, subgingival gel, emulsion, mouth rinse, mousse, foam, mouth spray, lozenge, chewable tablet, chewing gum, tooth whitening strips, floss and floss coatings, breath freshening dissolvable strips, unit-dose composition, fibrous composition, or denture care or adhesive product. The oral care composition may also be incorporated onto strips or films for direct application or attachment to oral surfaces, such as tooth whitening strips. Examples of emulsion compositions include the emulsions compositions of U.S. Patent No. 11,147,753, jammed emulsions, such as the jammed oil-in-water emulsions of U.S. Patent No. 11,096,874. Examples of unit-dose compositions include the unit-dose compositions of U.S. Patent Application Publication No. 2019/0343732.

The term "dentifrice composition", as used herein, includes tooth or subgingival -paste, gel, or liquid formulations unless otherwise specified. The dentifrice composition may be a single-phase composition or may be a combination of two or more separate dentifrice compositions. The dentifrice composition may be in any desired form, such as deep striped, surface striped, multilayered, having a gel surrounding a paste, or any combination thereof. Each dentifrice composition in a dentifrice comprising two or more separate dentifrice compositions may be contained in a physically separated compartment of a dispenser and dispensed side-by-side.

"Active and other ingredients" useful herein may be categorized or described herein by their cosmetic and/or therapeutic benefit or their postulated mode of action or function. However, it is to be understood that the active and other ingredients useful herein can, in some instances, provide more than one cosmetic and/or therapeutic benefit or function or operate *via* more than one mode of action. Therefore, classifications herein are made for the sake of convenience and are not intended to limit an ingredient to the particularly stated function(s) or activities listed.

The term "orally acceptable carrier" comprises one or more compatible solid or liquid excipients or diluents which are suitable for topical oral administration. By "compatible," as used herein, is meant that the components of the composition are capable of being commingled without interaction in a manner which would substantially reduce the composition's stability and/or efficacy. The carriers or excipients useful in embodiments of the present invention can include the usual and conventional components of mouthwashes or mouth rinses. Mouthwash or mouth rinse carrier materials typically include, but are not limited to one or more of water, alcohol, humectants, surfactants, and acceptance improving agents, such as flavoring, sweetening, coloring and/or cooling agents.

The term "substantially free" as used herein refers to the presence of no more than 0.05%, preferably no more than 0.01%, and more preferably no more than 0.001%, of an indicated material in a composition, by total weight of such composition.

The term "essentially free" as used herein means that the indicated material is not deliberately added to the composition, or preferably not present at analytically detectable levels. It is meant to include compositions whereby the indicated material is present only as an impurity of one of the other materials deliberately added.

The term "oral hygiene regimen" or "regimen" can be for the use of two or more separate and distinct treatment steps for oral health, e.g., toothpaste, mouth rinse, floss, toothpicks, spray, water irrigator, massager.

The term "total water content" as used herein means both free water and water that is bound by other ingredients in the oral care composition.

For the purpose of this description, the relevant molecular weight (MW) to be used is that of the material added when preparing the composition, e.g., if the chelant is a citrate species, which can be supplied as citric acid, sodium citrate or indeed other salt forms, the MW used is that of the particular salt or acid added to the composition but ignoring any water of crystallization that may be present.

While compositions and methods are described herein in terms of "comprising" various components or steps, the compositions and methods can also "consist essentially of" or "consist of" the various components or steps, unless stated otherwise.

As used herein, the word "or" when used as a connector of two or more elements is meant to include the elements individually and in combination; for example, X or Y, means X or Y or both.

As used herein, the articles "a" and "an" are understood to mean one or more of the material that is claimed or described, for example, "an oral care composition" or "a bleaching agent."

All measurements referred to herein are made at about 23 °C (i.e., room temperature) unless otherwise specified.

Generally, groups of elements are indicated using the numbering scheme indicated in the version of the periodic table of elements published in Chemical and Engineering News, 63(5), 27, 1985. In some instances, a group of elements can be indicated using a common name assigned to the group; for example, alkali metals for Group 1 elements, alkaline earth metals for Group 2 elements, and so forth.

Several types of ranges are disclosed in relation to embodiments of the present invention. When a range of any type is disclosed or claimed, the intent is to disclose or claim individually each possible number that such a range could reasonably encompass, including end points of the range as well as any sub-ranges and combinations of sub-ranges encompassed therein.

The oral care composition can be in any suitable form, such as a solid, liquid, powder, paste, or combinations thereof. The oral care composition can be dentifrice, tooth gel, subgingival gel, mouth rinse, mousse, foam, mouth spray, lozenge, chewable tablet, chewing gum, tooth whitening strips, floss and floss coatings, breath freshening dissolvable strips, or denture care or adhesive product. The components of the dentifrice composition can be incorporated into a film, a strip, a foam, or a fiber-based dentifrice composition.

The oral care composition can include a variety of active and inactive ingredients, such as, for example, but not limited to a hops extract, a dicarboxylic acid, a tin ion source, a calcium ion source, water, a fluoride ion source, zinc ion source, one or more polyphosphates, humectants, surfactants, other ingredients, and the like, as well as any combination thereof, as described below. The section headers below are provided for organization and convenience only. In some cases, a compound can fall within one or more sections. For example, stannous fluoride can be a tin compound and/or a fluoride compound. Additionally, oxalic acid, or salts thereof, can be a dicarboxylic acid, a polydentate ligand, and/or a whitening agent.

### Humulus lupulus

Oral care compositions of the present invention can comprise hops. The hops can comprise at least one hops compound from Formula I and/or Formula IV. The compound from Formula I and/or Formula IV can be provided by any suitable source, such as an extract from *Humulus lupulus* or Hops, *Humulus lupulus* itself, a synthetically derived compound, and/or salts, prodrugs, or other analogs thereof. The hops extract can comprise one or more hops alpha acids, one or more hops iso-alpha acids, one or more hops beta acids, one or more hops oils, one or more flavonoids, one or more solvents, and/or water. Suitable hops alpha acids (generically shown in Formula I) can include humulone (Formula II), adhumulone, cohumulone, posthumulone, prehumulone, and/or mixtures thereof. Suitable hops iso-alpha acids can include *cis*-isohumulone and/or *trans*-isohumulone. The isomerization of humulone into *trans*-isohumulone can be represented by Formula III.

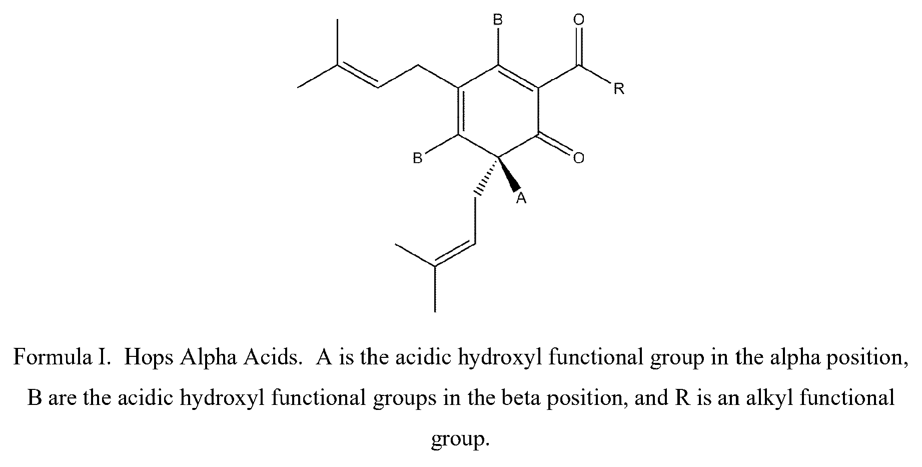

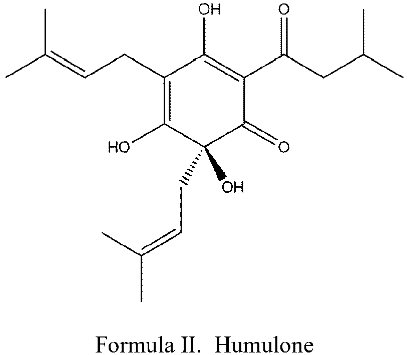

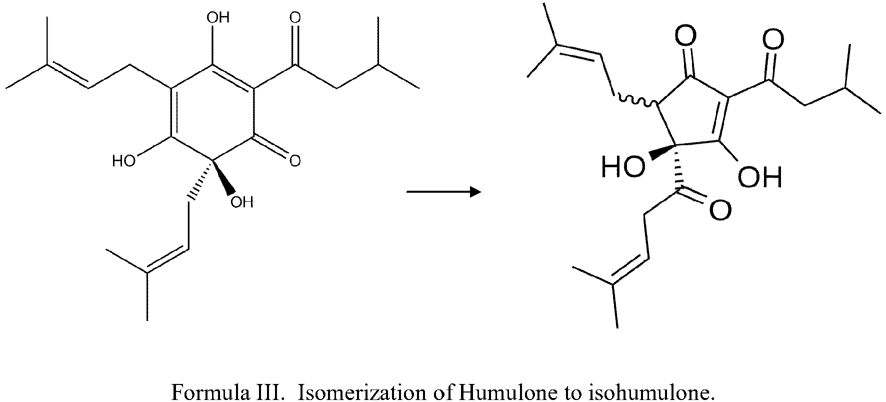

Suitable hops beta acids can include lupulone, adlupulone, colupulone, and/or mixtures thereof. A suitable hops beta acid can include a compound a described in Formula IV, V, VI, and/or VII.

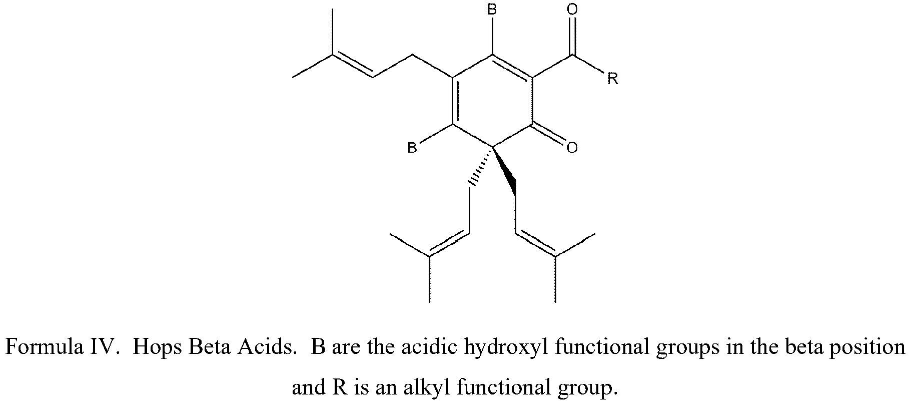

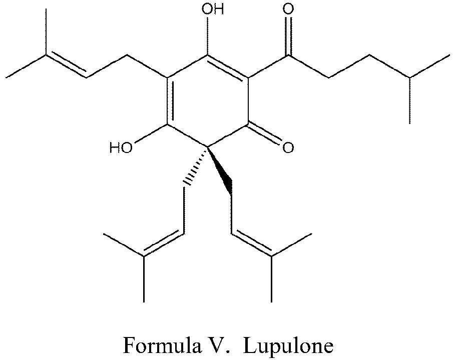

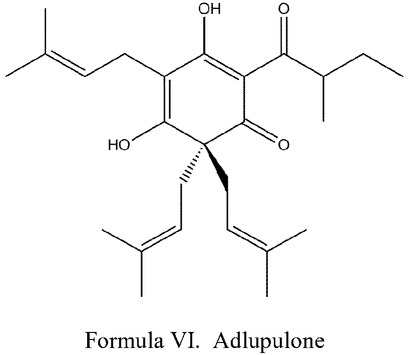

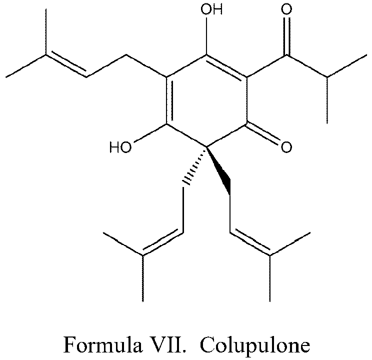

While hops alpha acids can demonstrate some antibacterial activity, hops alpha acids also have a bitter taste. The bitterness provided by hops alpha acids can be suitable for beer, but they are not suitable for use in oral care compositions. In contrast, hops beta acids can be associated with a higher antibacterial and/or anticaries activity, but not as bitter a taste. Thus, a hops extract with a higher proportion of beta acids to alpha acids than normally found in nature, can be suitable for use in oral care compositions for use as an antibacterial and/or anticaries agent.

A natural hops source can comprise from about 2% to about 12%, by weight of the hops source, of hops beta acids depending on the variety of hops. Hops extracts used in other contexts, such as in the brewing of beer, can comprise from about 15% to about 35%, by weight of the extract, of hops beta acids. The hops extract desired herein can comprise at least about 35%, at least about 40%, at least about 45%, from about 35% to about 95%, from about 40% to about 90%, or from about 45% to about 99%, of hops beta acids. The hops beta acids can be in an acidic form (i.e. with attached hydrogen atom(s) to the hydroxyl functional group(s)) or as a salt form.

A suitable hops extract is described in detail in U.S. Patent No. 7,910,140, which is herein incorporated by reference in its entirety. The hops beta acids desired can be non-hydrogenated, partially hydrogenated by a non-naturally occurring chemical reaction, or hydrogenated by a non-naturally occurring chemical reaction. The hops beta acid can be essentially free of or substantially free of hydrogenated hops beta acid and/or hops acid. A non-naturally occurring chemical reaction is a chemical reaction that was conducted with the aid of chemical compound not found within *Humulus lupulus*, such as a chemical hydrogenation reaction conducted with high heat not normally experienced by *Humulus lupulus* in the wild and/or a metal catalyst.

A natural hops source can comprise from about 2% to about 12%, by weight of the hops source, of hops alpha acids. Hops extracts used in other contexts, such as in the brewing of beer, can comprise from about 15% to about 35%, by weight of the extract, of hops alpha acids. The hops extract desired herein can comprise less than about 10%, less than about 5%, less than about 1%, or less than about 0.5%, by weight of the extract, of hops alpha acids.

Hops oils can include terpene hydrocarbons, such as myrcene, humulene, caryophyllene, and/or mixtures thereof. The hops extract desired herein can comprise less than 5%, less than 2.5%, or less than 2%, by weight of the extract, of one or more hops oils.

Flavonoids present in the hops extract can include xanthohumol, 8-prenylnaringenin, isoxanthohumol, and/or mixtures thereof. The hops extract can be substantially free of, essentially free of, free of, or have less than 250 ppm, less than 150 ppm, and/or less than 100 ppm of one or more flavonoids.

As described in U.S. Patent No. 5,370,863, hops acids have been previously added to oral care compositions. However, the oral care compositions taught by U.S. Patent No. 5,370,863 only included up to 0.01%, by weight of the oral care composition. While not wishing to be bound by theory, it is believed that U.S. Patent No. 5,370,863 could only incorporate a low amount of hops acids because of the bitterness of hops alpha acids. A hops extract with a low level of hops alpha acids would not have this concern.

The hops compound can be combined with or free from an extract from another plant, such as a species from genus *Magnolia*, *Garcinia mangostana L.*, or *Zizyphus joazeiro*. The oral care composition may comprise less than about 0.5%, less than about 0.1%, or less than about 0.01% of an extract from a plant other than hops, such as a species from genus *Magnolia*, *Garcinia mangostana L.*, or *Zizyphus joazeiro*. The hops compounds can be combined with or free from a nonionic halogenated diphenyl ether, such as triclosan.

The oral care composition can comprise from about 0.01% to about 10%, greater than 0.01% to about 10%, from about 0.05%, to about 10%, from about 0.1% to about 10%, from about 0.2% to about 10%, from about 0.2% to about 10%, from about 0.2% to about 5%, from about 0.25% to about 2%, from about 0.05% to about 2%, or from greater than 0.25% to about 2%, of hops, such as hops beta acid, as described herein. The hops, such as the hops beta acid, can be provided by a suitable hops extract, the hops plant itself, or a synthetically derived compound. The hops, such as hops beta acid, can be provided as neutral, acidic compounds, and/or as salts with a suitable counter ion, such as sodium, potassium, ammonia, or any other suitable counter ion.

The hops can be provided by a hops extract, such as an extract from *Humulus lupulus* with at least 35%, by weight of the extract, of hops beta acid and less than 1%, by weight of the hops extract, of hops alpha acid. The oral care composition can comprise 0.01% to about 10%, greater than 0.01% to about 10%, from about 0.05%, to about 10%, from about 0.1% to about 10%, from about 0.2% to about 10%, from about 0.2% to about 10%, from about 0.2% to about 5%, from about 0.25% to about 2%, from about 0.05% to about 2%, or from greater than 0.25% to about 2%, of hops extract, as described herein.

### Dicarboxylic Acid

The oral care composition comprises dicarboxylic acid. The dicarboxylic acid comprises a compound with two carboxylic acid functional groups. The dicarboxylic acid can comprise a compound or salt thereof defined by Formula VIII-A, Formula VIII-B, and/or Formula VIII-C.

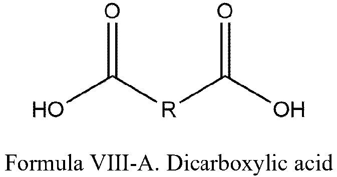

R can be null, alkyl, alkenyl, allyl, phenyl, benzyl, acetyl, aliphatic, aromatic, polyethylene glycol, polymer, O, N, P, or combinations thereof. R can also be additionally functionalized with one or more functional groups, such as -OH, -NH₂, and/or alkyl, alkenyl, aromatic, or combinations thereof.

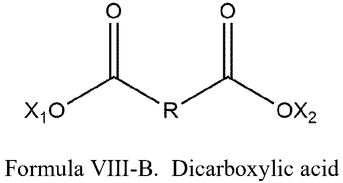

R can be null, alkyl, alkenyl, allyl, phenyl, benzyl, acetyl, aliphatic, aromatic, polyethylene glycol, polymer, O, N, P, or combinations thereof. R can also be additionally functionalized with one or more functional groups, such as -OH, -NH₂, and/or alkyl, alkenyl, aromatic, or combinations thereof.

X₁ and X₂ can independently be H, alkali metal, alkali earth metal, transition metal, or combinations thereof. Suitable alkali metals include lithium, sodium, potassium, or combinations thereof. Suitable alkali earth metals include magnesium, calcium, barium, or combinations thereof. Suitable transitional metals include titanium, chromium, iron, nickel, copper, zinc, tin, gold, silver, or combinations thereof.

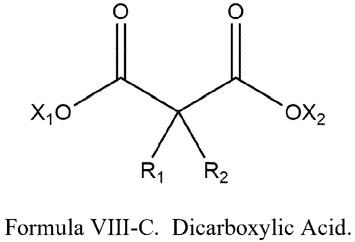

R₁ can be null, alkyl, alkenyl, allyl, phenyl, benzyl, acetyl, aliphatic, aromatic, polyethylene glycol, polymer, O, N, P, or combinations thereof. R can also be additionally functionalized with one or more functional groups, such as -OH, -NH₂, and/or alkyl, alkenyl, aromatic, or combinations thereof.

X₁ and X₂ can independently be H, alkali metal, alkali earth metal, transition metal, or combinations thereof. Suitable alkali metals include lithium, sodium, potassium, or combinations thereof. Suitable alkali earth metals include magnesium, calcium, barium, or combinations thereof. Suitable transitional metals include titanium, chromium, iron, nickel, copper, zinc, tin, gold, silver, or combinations thereof.

The dicarboxylic acid can be added to a formulation as a neutral acid (as shown in Formula VIII-A) or as a dicarboxylate monosalt (where one of the carboxylic acid functional groups is a salt and the other is neutral), a dicarboxylate disalt (where both of the carboxylic acid functional groups are salts), or combinations thereof. Additionally, as is well known to a person of ordinary skill in the art, whether or not that one or both of the carboxylic acid functional groups of the dicarboxylic acid are neutral or charged in solution, can be influenced by the pH of the solution. For example, a neutral dicarboxylic acid can be added to an aqueous solution and one or two protons from the two carboxylic acid functional groups can be removed if the pH is lower than the pKa of the carboxylic acid functional group, as shown below in Formula VIII-D.

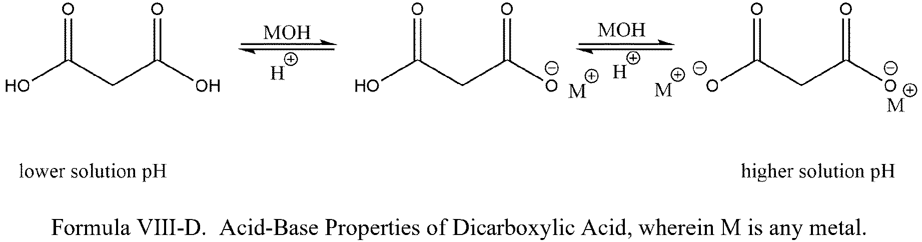

The dicarboxylic acid can comprise oxalic acid, malonic acid, succinic acid, glutaric acid, adipic acid, pimelic acid, suberic acid, azerlaic acid, sebacic acid, undecanedioic acid, dodecanedioic acid, brassylic acid, thapsic acid, japanic acid, phellogenic acid, equisetolic acid, malic acid, maleic acid, tartaric acid, phthalic acid, methylmalonic acid, dimethylmalonic acid, tartronic acid, mesoxalic acid, dihydroxymalonic acid, dihydroxymalonic acid, fumaric acid, terephthalic acid, glutaric acid, salts thereof, or combinations thereof. The dicarboxylic acid can comprise suitable salts of dicarboxylic acid, such as, for example, when the dicarboxylic acid includes a salt of oxalic acid: monoalkali metal oxalate, dialkali metal oxalate, monopotassium monohydrogen oxalate, dipotassium oxalate, monosodium monohydrogen oxalate, disodium oxalate, titanium oxalate, and/or other metal salts of oxalate. The dicarboxylic acid can also include hydrates of the dicarboxylic acid and/or a hydrate of a salt of the dicarboxylic acid.

Suitable dicarboxylic acid compounds include malonic acid, methylmalonic acid, tartronic acid, malic acid, dimethylmalonic acid, mesoxalic acid, dihydroxymalonic acid, oxalic acid, salts thereof, or combinations thereof. These dicarboxylic acid compounds are particularly suitable as these compounds have been shown to have an unexpectedly high whitening benefit. While not wishing to be bound by theory, it is believed that particular dicarboxylic acid compounds have an unexpectedly high affinity to certain cationic crosslinking agents typically found in the colored matrix on the oral hard tissue surfaces, thereby resulting in the removal of stain from the surface.

Suitable dicarboxylic acid compounds include dicarboxylic acids described by Formula VIII-A, wherein R is null, comprises a methylene or ethylene with one or two substitutions, and/or an acetyl group.

Without being bound by theory, it is hypothesized that the whitening efficacy of the dicarboxylics acids and their corresponding anions is driven by the ability of the dicarboxylic acid to reach and remove cationic bridges between chromophores and the tooth surface as well as chromophores and the pellicle proteins.

### Fluoride

The oral care composition can comprise fluoride, which can be provided by a fluoride ion source. The fluoride ion source can comprise one or more fluoride containing compounds, such as stannous fluoride, sodium fluoride, titanium fluoride, calcium fluoride, calcium phosphate silicate fluoride, potassium fluoride, amine fluoride, sodium monofluorophosphate, zinc fluoride, and/or mixtures thereof.

The fluoride ion source and the tin ion source can be the same compound, such as for example, stannous fluoride, which can generate tin ions and fluoride ions. Additionally, the fluoride ion source and the tin ion source can be separate compounds, such as when the tin ion source is stannous chloride and the fluoride ion source is sodium monofluorophosphate or sodium fluoride.

The fluoride ion source and the zinc ion source can be the same compound, such as for example, zinc fluoride, which can generate zinc ions and fluoride ions. Additionally, the fluoride ion source and the zinc ion source can be separate compounds, such as when the zinc ion source is zinc phosphate and the fluoride ion source is stannous fluoride.

The fluoride ion source can be essentially free of, or free of stannous fluoride. Thus, the oral care composition can comprise sodium fluoride, potassium fluoride, amine fluoride, sodium monofluorophosphate, zinc fluoride, and/or mixtures thereof.

The oral care composition can comprise a fluoride ion source capable of providing from about 50 ppm to about 5000 ppm, and preferably from about 500 ppm to about 3000 ppm of free fluoride ions. To deliver the desired amount of fluoride ions, the fluoride ion source may be present in the oral care composition at an amount of from about 0.0025% to about 5%, from about 0.01% to about 10%, from about 0.2% to about 1%, from about 0.5% to about 1.5%, or from about 0.3% to about 0.6%, by weight of the oral care composition. Alternatively, the oral care composition can comprise less than 0.1%, less than 0.01%, be essentially free of, be substantially free of, or be free of a fluoride ion source.

### Metal

The oral care composition, as described herein, can comprise metal, which can be provided by a metal ion source comprising one or more metal ions. The metal ion source can comprise or be in addition to the tin ion source and/or the zinc ion source, as described herein. Suitable metal ion sources include compounds with metal ions, such as, but not limited to Sn, Zn, K, Cu, Mn, Mg, Sr, Ti, Fe, Mo, B, Ba, Ce, Al, In and/or mixtures thereof. The metal ion source can be any compound with a suitable metal and any accompanying ligands and/or anions.

Suitable ligands and/or anions that can be paired with metal ion sources include, but are not limited to acetate, ammonium sulfate, benzoate, bromide, borate, carbonate, chloride, citrate, gluconate, glycerophosphate, hydroxide, iodide, oxalate, oxide, propionate, D-lactate, DL-lactate, orthophosphate, pyrophosphate, sulfate, nitrate, tartrate, and/or mixtures thereof.

The oral care composition can comprise from about 0.01% to about 10%, from about 1% to about 5%, or from about 0.5% to about 15% of metal and/or a metal ion source.

### Tin

An oral care composition according to embodiments of the present invention can comprise tin, which can be provided by a tin ion source. The tin ion source can be any suitable compound that can provide tin ions in an oral care composition and/or deliver tin ions to the oral cavity when the oral care composition is applied to the oral cavity. The tin ion source can comprise one or more tin containing compounds, such as stannous fluoride, stannous chloride, stannous bromide, stannous iodide, stannous oxide, stannous oxalate, stannous sulfate, stannous sulfide, stannic fluoride, stannic chloride, stannic bromide, stannic iodide, stannic sulfide, and/or mixtures thereof. Tin ion source can comprise stannous fluoride, stannous chloride, and/or mixture thereof. The tin ion source can also be a fluoride-free tin ion source, such as stannous chloride.

The oral care composition can comprise from about 0.0025% to about 5%, from about 0.01% to about 10%, from about 0.2% to about 1%, from about 0.4% to about 1%, or from about 0.3% to about 0.6%, by weight of the oral care composition, of tin and/or a tin ion source. Alternatively, the oral care composition can be essentially free of, substantially free of, or free of tin.

### Antibacterial Agents

The oral care composition can comprise one or more antibacterial agents. Suitable antibacterial agents include any molecule that provides antibacterial activity in the oral cavity. Suitable antibacterial agents include hops acids, tin ion sources, benzyl alcohol, sodium benzoate, menthylglycyl acetate, menthyl lactate, L-menthol, o-neomenthol, chlorophyllin copper complex, phenol, oxyquinoline, and/or combinations thereof.

The oral care composition can comprise from about 0.01% to about 10%, from about 1% to about 5%, or from about 0.5% to about 15% of an antibacterial agent.

### Bioactive Materials

The oral care composition can also include bioactive materials suitable for the remineralization of a tooth. Suitable bioactive materials include bioactive glasses, Novamin^{™}, Recaldent^{™}, hydroxyapatite, one or more amino acids, such as, for example, arginine, citrulline, glycine, lysine, or histidine, or combinations thereof. Suitable examples of compositions comprising arginine are found in U.S. Patent No. 4,154,813 and 5,762,911, which are herein incorporated by reference in their entirety. Other suitable bioactive materials include any calcium phosphate compound. Other suitable bioactive materials include compounds comprising a calcium source and a phosphate source.

Amino acids are organic compounds that contain an amine functional group, a carboxyl functional group, and a side chain specific to each amino acid. Suitable amino acids include, for example, amino acids with a positive or negative side chain, amino acids with an acidic or basic side chain, amino acids with polar uncharged side chains, amino acids with hydrophobic side chains, and/or combinations thereof. Suitable amino acids also include, for example, arginine, histidine, lysine, aspartic acid, glutamic acid, serine, threonine, asparagine, glutamine, cysteine, selenocysteine, glycine, proline, alanine, valine, isoleucine, leucine, methionine, phenylalanine, tyrosine, tryptophan, citrulline, ornithine, creatine, diaminobutonic acid, diaminoproprionic acid, salts thereof, and/or combinations thereof.

Bioactive glasses are comprising calcium and/or phosphate which can be present in a proportion that is similar to hydroxyapatite. These glasses can bond to the tissue and are biocompatible. Bioactive glasses can include a phosphopeptide, a calcium source, phosphate source, a silica source, a sodium source, and/or combinations thereof.

The oral care composition can comprise from about 0.01% to about 20%, from about 0.1% to about 10%, or from about 1% to about 10 % of a bioactive material by weight of the oral care composition.

### Zinc

The oral care composition can comprise zinc, which can be provided by a zinc ion source. The zinc ion source can comprise one or more zinc containing compounds, such as zinc fluoride, zinc lactate, zinc oxide, zinc phosphate, zinc chloride, zinc acetate, zinc hexafluorozirconate, zinc sulfate, zinc tartrate, zinc gluconate, zinc citrate, zinc malate, zinc glycinate, zinc pyrophosphate, zinc metaphosphate, zinc oxalate, and/or zinc carbonate. The zinc ion source can be a fluoride-free zinc ion source, such as zinc phosphate, zinc oxide, and/or zinc citrate.

The zinc and/or zinc ion source may be present in the total oral care composition at an amount of from about 0.01% to about 10%, from about 0.2% to about 1%, from about 0.4% to about 1%, from about 0.5% to about 1.5%, or from about 0.3% to about 0.6%, by weight of the oral care composition. Alternatively, the oral care composition can be essentially free of, substantially free of, or free of zinc. In an embodiment, the oral care composition can be essentially free of, substantially free of, or free of soluble zinc.

### Potassium

The oral care composition can comprise potassium, which can be provided by a potassium ion source. The potassium ion source can comprise one or more potassium containing compounds, such as potassium nitrate, potassium fluoride, potassium chloride, or combinations thereof.

The oral care composition can comprise from about 0.01% to about 10%, from about 0.2% to about 1%, from about 0.4% to about 1 %, or from about 0.3% to about 0.6%, by weight of the oral care composition, of potassium and/or potassium ion source. Alternatively, the oral care composition can be essentially free of, substantially free of, or free of potassium.

### Quaternary Ammonium Compound

The oral care composition can include quaternary ammonium compound. The quaternary ammonium compounds in the compositions of embodiments of the present invention can include those in which one or two of the substitutes on the quaternary nitrogen has a carbon chain length (typically alkyl group) from about 8 to about 20, typically from about 10 to about 18 carbon atoms while the remaining substitutes (typically alkyl or benzyl group) have a lower number of carbon atoms, such as from about 1 to about 7 carbon atoms, typically methyl or ethyl groups. Cetylpyridinium chloride, cetyl pyridinium fluoride, tetradecylpyridinium chloride, N-tetradecyl-4-ethyl pyridinium chloride, domiphen bromide, benzalkonium chloride, benzethonium chloride, methyl benzethonium chloride, dodecyl trimethyl ammonium bromide, dodecyl dimethyl (2-phenoxyethyl) ammonium bromide, benzyl dimethoxystearyl ammonium chloride, quaternized 5-amino-1,3-bis(2-ethyl-hexyl)-5-methyl hexa hydropyrimidine, lauryl trimethylammonium chloride, cocoalkyl trimethylammonium chloride, cetyl trimethylammonium bromide, di-isobutylphenoxyethyl-dimethylbenzylammonium chloride, dodecyl trimethyl ammonium bromide, are exemplary of typical quaternary ammonium antimicrobial agents. Other compounds are bis[4-(R-amino)-1-pyridinium] alkanes as disclosed in U.S. No. 4,206,215 to Bailey. The pyridinium compounds are the preferred quaternary ammonium compounds, particularly preferred being cetylpyridinium, or tetradecylpyridinium halide salts (i.e., chloride, bromide, fluoride and iodide). Particularly preferred are cetylpyridinium chloride and fluoride salts.

The oral care composition can comprise at least about 0.025%, at least about 0.035%, at least about 0.045% to about 1.0%, from about 0.025% to about 1%, or from about 0.01% to about 10%, by weight of the composition, of the quaternary ammonium compound. Alternatively, the oral care composition can be essentially free of, substantially free of, or free of a quaternary ammonium compound.

### pH

The pH of the oral care compositions as described herein can be from about 4 to about 10, from about 4.5 to about 9, from about 7 to about 10, greater than 7 to about 10, greater than 8 to about 10, greater than 7, greater than 7.5, greater than 8, greater than 9, from about 8.5 to about 10, from about 4 to about 7, from about 4 to about 6, from about 4.5 to about 6.5, from about 4.5 to about 5.5, from about 4 to less than 5.5, from about 4.5 to less than 5.5, greater than 4 to less than 5, greater than 4 to about 4.9, from about 4.9, from about 4 to about 5.4, from about 4 to about 5.3, from about 4 to about 5.2, from about 4 to about 5.1, from about 4 to about 5, from about 4 to about 4.9, from about 4 to about 4.8, from about 4 to about 4.7, or from about 4.8 to about 5.3. The pH of a mouth rinse solution can be determined as the pH of the neat solution. The pH of a dentifrice composition can be determined as a slurry pH, which is the pH of a mixture of the dentifrice composition and water, such as a 1:4, 1:3, or 1:2 mixture of the dentifrice composition and water.

If the oral care composition comprises one or more dicarboxylic acids, a preferred pH is below about 7 or below about 6 due to the pKa of the dicarboxylic acid. While not wishing to be bound by theory, it is believed that the dicarboxylic acid displays unique behavior when the pH is below about 7 or below about 6, but surfaces in the oral cavity can also be sensitive to a low pH. Additionally, at pH values above about pH 7, the metal ion source can react with water and/or hydroxide ions to form insoluble metal oxides and/or metal hydroxides. The formation of these insoluble compounds can limit the ability of dicarboxylates to stabilize metal ions in oral care compositions and/or can limit the interaction of dicarboxylates with target metal ions in the oral cavity.

Additionally, at pH values less than 4, the potential for demineralization is greatly increased. Consequently, the oral care compositions comprising dicarboxylic acid, as described herein, can preferably have a pH from about 4 to about 7, from about 4 to about 6, from about 4.5 to about 6.5, from about 4 to about 5, from about 4 to less than 5, from about 4 to about 4.9, or from about 4.5 to less than 5.5 to minimize metal hydroxide/metal oxide formation and any increased demineralization in the oral cavity.

The pH of the oral care composition, as described herein, can be measured either immediately upon mixing, or upon aging the composition by placing the oral care composition at ambient or accelerated temperature and humidity conditions, such as including measuring the pH at a temperature of 25 °C, 30 °C and/or 40 °C with a 30%, 60% and/or 75% relative humidity for about 28 days or longer prior to measuring the pH.

### Buffering Agents

The oral care composition can comprise one or more buffering agents. Buffering agents, as used herein, refer to agents that can be used to adjust the slurry pH of the oral care compositions. The buffering agents include alkali metal hydroxides, carbonates, sesquicarbonates, borates, silicates, phosphates, imidazole, carboxylates, and mixtures thereof. Specific buffering agents include monosodium phosphate, trisodium phosphate, sodium hydroxide, potassium hydroxide, alkali metal carbonate salts, sodium carbonate, imidazole, pyrophosphate salts, citric acid, and sodium citrate. The oral care composition can comprise one or more buffering agents each at a level of from about 0.1 % to about 30%, from about 1% to about 10%, or from about 1.5% to about 3%, by weight of the present composition.

### Polyphosphate

The oral care composition can comprise polyphosphate, which can be provided by a polyphosphate source. A polyphosphate source can comprise one or more polyphosphate molecules. Polyphosphates are a class of materials obtained by the dehydration and condensation of orthophosphate to yield linear and cyclic polyphosphates of varying chain lengths. Thus, polyphosphate molecules are generally identified with an average number (n) of polyphosphate molecules, as described below. A polyphosphate is generally understood to consist of two or more phosphate molecules arranged primarily in a linear configuration, although some cyclic derivatives may be present.

Preferred polyphosphates are those having an average of two or more phosphate groups so that surface adsorption at effective concentrations produces sufficient non-bound phosphate functions, which enhance the anionic surface charge as well as hydrophilic character of the surfaces. Preferred polyphosphates include linear polyphosphates having the formula: XO(XPO₃)ₙX, wherein X is sodium, potassium, ammonium, or any other alkali metal cations and n averages from about 2 to about 21. Alkali earth metal cations, such as calcium, are not preferred because they tend to form insoluble fluoride salts from aqueous solutions comprising a fluoride ions and alkali earth metal cations. Thus, the oral care compositions disclosed herein can be free of, essentially free of, or substantially free of calcium pyrophosphate.

Some examples of suitable polyphosphate molecules include, for example, pyrophosphate (n=2), tripolyphosphate (n=3), tetrapolyphosphate (n=4), sodaphos polyphosphate (n=6), hexaphos polyphosphate (n=13), benephos polyphosphate (n=14), hexametaphosphate (n=21), which is also known as Glass H. Polyphosphates can include those polyphosphate compounds manufactured by FMC Corporation, ICL Performance Products, and/or Astaris.

The oral care composition can comprise from about 0.01% to about 15%, from about 0.1% to about 10%, from about 0.5% to about 5%, from about 1 to about 20%, or about 10% or less, by weight of the oral care composition, of the polyphosphate source. Alternatively, the oral care composition can be essentially free of, substantially free of, or free of polyphosphate.

### Surfactants

The oral care composition can comprise one or more surfactants. The surfactants can be used to make the compositions more cosmetically acceptable. The surfactant is preferably a detersive material which imparts to the composition detersive and foaming properties. Suitable surfactants are safe and effective amounts of anionic, cationic, nonionic, zwitterionic, amphoteric and betaine surfactants.

Suitable anionic surfactants include, for example, the water soluble salts of alkyl sulfates having from 8 to 20 carbon atoms in the alkyl radical and the water-soluble salts of sulfonated monoglycerides of fatty acids having from 8 to 20 carbon atoms. Sodium lauryl sulfate (SLS) and sodium coconut monoglyceride sulfonates are examples of anionic surfactants of this type. Other suitable anionic surfactants include sarcosinates, such as sodium lauroyl sarcosinate, taurates, sodium lauryl sulfoacetate, sodium lauroyl isethionate, sodium laureth carboxylate, and sodium dodecyl benzene sulfonate. Combinations of anionic surfactants can also be employed.

Another suitable class of anionic surfactants are alkyl phosphates. The surface active organophosphate agents can have a strong affinity for enamel surface and have sufficient surface binding propensity to desorb pellicle proteins and remain affixed to enamel surfaces. Suitable examples of organophosphate compounds include mono-, di- or triesters represented by the general structure below: 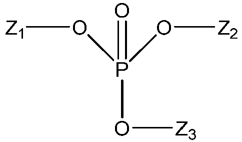 wherein Z₁, Z₂, or Z₃ may be identical or different with at least one being an organic moiety. Z₁, Z₂, or Z₃ can be selected from linear or branched, alkyl or alkenyl group of from 1 to 22 carbon atoms, optionally substituted by one or more phosphate groups; alkoxylated alkyl or alkenyl, (poly)saccharide, polyol or polyether group. Some other agents include alkyl or alkenyl phosphate esters represented by the following structure: 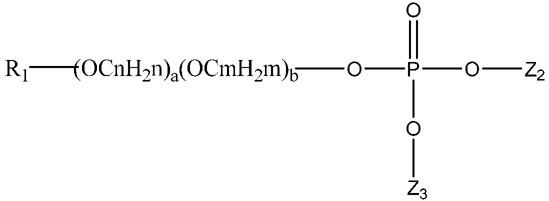 wherein R₁ represents a linear or branched, alkyl or alkenyl group of from 6 to 22 carbon atoms, optionally substituted by one or more phosphate groups; n and m, are individually and separately, 2 to 4, and a and b, individually and separately, are 0 to 20; Z and Z may be identical or different, each represents hydrogen, alkali metal, ammonium, protonated alkyl amine or protonated functional alkylamine, such as analkanolamine, or a R-(OCH2)(OCH) - group. Examples of suitable agents include alkyl and alkyl (poly)alkoxy phosphates such as lauryl phosphate; PPGS ceteareth-10 phosphate; laureth-1 phosphate; laureth-3 phosphate; laureth-9 phosphate; trilaureth-4 phosphate; C₁₂₋₁₈ PEG 9 phosphate: and sodium dilaureth-10 phosphate. The alkyl phosphate can be polymeric. Examples of polymeric alkyl phosphates include those containing repeating alkoxy groups as the polymeric portion, in particular 3 or more ethoxy, propoxy isopropoxy or butoxy groups.

Other suitable anionic surfactants are sarcosinates, isethionates and taurates, especially their alkali metal or ammonium salts. Examples include: lauroyl sarcosinate, myristoyl sarcosinate, palmitoyl sarcosinate, stearoyl sarcosinate oleoyl sarcosinate, or combinations thereof.

Other suitable anionic surfactants include sodium or potassium alkyl sulfates, such as sodium lauryl sulfate, acyl isethionates, acyl methyl isethionates, alkyl ether carboxylates, acyl alaninates, acyl gulatames, acyl glycinates, acyl sarconsinates, sodium methyl acyl taurates, sodium laureth sulfosuccinates, alpha olefin sulfonates, alkyl benze sulfonates, sodium lauroyl lactylate, sodium laurylglucosides hydroxypropyl sulfonate, and/or combinations.

A suitable taurate surfactant is represented by the following formula: 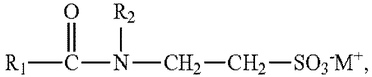 wherein R₁ is a saturated or unsaturated, straight, or branched alkyl chain with 6 to 18 C atoms; R₂ is H or methyl, and M is H, sodium, or potassium. Preferably, the R₁ is a saturated or unsaturated, straight, or branched alkyl chain with 8 to 18 C atoms. Optionally but preferably, the taurate surfactant comprises one or more selected from the group consisting of potassium cocoyl taurate, potassium methyl cocoyl taurate, sodium caproyl methyl taurate, sodium cocoyl taurate, sodium lauroyl taurate, sodium methyl cocoyl taurate, sodium methyl lauroyl taurate, sodium methyl myristoyl taurate, sodium methyl oleoyl taurate, and combinations thereof.

Zwitterionic or amphoteric surfactants useful herein include derivatives of aliphatic quaternary ammonium, phosphonium, and Sulfonium compounds, in which the aliphatic radicals can be straight chain or branched, and one of the aliphatic substituents contains from 8 to 18 carbon atoms and one contains an anionic water-solubilizing group, e.g., carboxy, sulfonate, sulfate, phosphate or phosphonate. Suitable betaine surfactants are disclosed in U.S. Pat. No. 5,180,577. Typical alkyl dimethyl betaines include decyl betaine or 2-(N-decyl-N,N-dimethylammonio) acetate, coco-betaine or 2-(N-coco-N,N-dimethyl ammonio)acetate, myristyl betaine, palmityl betaine, lauryl betaine, cetyl betaine, cetyl betaine, stearyl betaine, *etc.* The amidobetaines can be exemplified by cocoamidoethyl betaine, cocoamidopropyl betaine (CADB), and lauramidopropyl betaine. Other suitable amphoteric surfactants include betaines, sultaines, sodium laurylamphoacetates, alkylamphodiacetates, and/or combinations thereof.

Suitable cationic surfactants include, for example, derivatives of quaternary ammonium compounds having one long alkyl chain containing from 8 to 18 carbon atoms such as lauryl trimethylammonium chloride; cetyl pyridinium chloride; cetyl trimethyl-ammonium bromide; cetyl pyridinium fluoride or combinations thereof.

Suitable nonionic surfactants include, for example, compounds produced by the condensation of alkylene oxide groups (hydrophilic in nature) with an organic hydrophobic compound which may be aliphatic or alkylaromatic in nature. Examples of suitable nonionic surfactants can include the Pluronics^{®} which are poloxamers, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and combinations of such materials. Other suitable non-ionic surfactants includes alkyl glucamides, alkyl glucosides, and/or combinations thereof.

The one or more surfactants can also include one or more natural and/or naturally derived surfactants. Natural surfactants can include surfactants that are derived from natural products and/or surfactants that are minimally or not processed. Natural surfactants can include hydrogenated, non-hydrogenated, or partially hydrogenated vegetable oils, olus oil, passiflora incarnata oil, candelilla cera, coco-caprylate, caprate, dicaprylyl ether, lauryl alcohol, myristyl myristate, dicaprylyl ether, caprylic acid, caprylic ester, octyl decanoate, octyl octanoate, undecane, tridecane, decyl oleate, oleic acid decylester, cetyl palmitate, stearic acid, palmitic acid, glyceryl stearate, hydrogenated, non-hydrogenated, or partially hydrogenated vegetable glycerides, Polyglyceryl-2 dipolyhydroxystearate, cetearyl alcohol, sucrose polystearate, glycerin, octadodecanol, hydrolyzed, partially hydrolyzed, or non-hydrolyzed vegetable protein, hydrolyzed, partially hydrolyzed, or non-hydrolyzed wheat protein hydrolysate, polyglyceryl-3 diisostearate, glyceryl oleate, myristyl alcohol, cetyl alcohol, sodium cetearyl sulfate, cetearyl alcohol, glyceryl laurate, capric triglyceride, coco-glycerides, lectithin, dicaprylyl ether, xanthan gum, sodium coco-sulfate, ammonium lauryl sulfate, sodium cocoyl sulfate, sodium cocoyl glutamate, polyalkylglucosides, such as decyl glucoside, cetearyl glucoside, cetyl stearyl polyglucoside, coco-glucoside, and lauryl glucoside, and/or combinations thereof. Natural surfactants can include any of the Natrue ingredients marketed by BASF, such as, for example, CegeSoft^{®}, Cetiol^{®}, Cutina^{®}, Dehymuls^{®}, Emulgade^{®}, Emulgin^{®}, Eutanol^{®}, Gluadin^{®}, Lameform^{®}, LameSoft^{®}, Lanette^{®}, Monomuls^{®}, Myritol^{®}, Plantacare^{®}, Plantaquat^{®}, Platasil^{®}, Rheocare^{®}, Sulfopon^{®}¸ Texapon^{®}, and/or combinations thereof.

Other specific examples of surfactants include sodium lauryl sulfate, sodium lauryl isethionate, sodium lauroyl methyl isethionate, sodium cocoyl glutamate, sodium dodecyl benzene sulfonate, alkali metal or ammonium salts of lauroyl sarcosinate, myristoyl sarcosinate, palmitoyl sarcosinate, stearoyl sarcosinate and oleoyl sarcosinate, polyoxyethylene sorbitan monostearate, isostearate and laurate, sodium lauryl sulfoacetate, N-lauroyl sarcosine, the sodium, potassium, and ethanolamine salts of N-lauroyl, N-myristoyl, or N-palmitoyl sarcosine, polyethylene oxide condensates of alkyl phenols, cocoamidopropyl betaine, lauramidopropyl betaine, palmityl betaine, sodium cocoyl glutamate, and the like. Additional surfactants desired include fatty acid salts of glutamate, alkyl glucoside, salts of taurates, betaines, caprylates, and/or mixtures thereof. The oral care composition can also be sulfate free. The oral care composition can comprise one or more surfactants each at a level from about 0.01% to about 15%, from about 0.3% to about 10%, or from about 0.3% to about 2.5%, by weight of the oral care composition, of neat surfactant.

In some embodiments, an oral care composition may include one or more surfactants selected from the group consisting of sodium lauryl sulfate, cocamidopropyl betaine, sodium cocoyl glutamate, sodium methyl cocoyl taurate, lauryl glucoside, and poloxamer. In some embodiments, an oral care composition may include one or more surfactants consisting essentially of sodium lauryl sulfate, cocamidopropyl betaine, sodium cocoyl glutamate, sodium methyl cocoyl taurate, lauryl glucoside, poloxamer, or combinations thereof. In some embodiments, an oral care composition may include one or more surfactants consisting of sodium lauryl sulfate, cocamidopropyl betaine, sodium cocoyl glutamate, sodium methyl cocoyl taurate, lauryl glucoside, poloxamer, or combinations thereof. In some embodiments, the oral care composition may include one or more surfactants that consist essentially of sodium lauryl sulfate, cocamidopropyl betaine, sodium methyl cocoyl taurate, or a combination thereof.

In some embodiments, the oral care composition may include a main surfactant and a co-surfactant, where the main surfactant concentration is greater than the concentration of the co-surfactant. In some embodiments where the oral care composition includes sodium cocoyl glutamate, lauryl glucoside, or poloxamer as a surfactant, then the oral care composition comprises at least two surfactants. The ratio of the surfactant to co-surfactant can be about 1:0.25, or about 1:0.33, or about 1:0.4, or about 1:0.5, or about 1:0.6, or about 1:0.75, or about 1:0.9, or about 1:1.

In some embodiments where an oral care composition includes a single surfactant, the single surfactant does not comprise sodium cocoyl glutamate, lauryl glucoside, or poloxamer. In some embodiments where the oral care composition includes a single surfactant, the single surfactant may include sodium lauryl sulfate, cocamidopropyl betaine, or sodium methyl cocoyl taurate. In some embodiments, the oral care composition may be essentially free of, substantially free of, or free of sodium cocoyl glutamate, lauryl glucoside, or poloxamer.

### Monodentate Ligand

The oral care composition can comprise monodentate ligand having a molecular weight (MW) of less than 1000 g/mol. A monodentate ligand has a single functional group that can interact with the central atom, such as a tin ion. The monodentate ligand must be suitable for the use in oral care composition, which can be include being listed in Generally Regarded as Safe (GRAS) list with the United States Food and Drug Administration or other suitable list in a jurisdiction of interest.

The monodentate ligand, as described herein, can include a single functional group that can chelate to, associate with, and/or bond to tin. Suitable functional groups that can chelate to, associate with, and/or bond to tin include carbonyl, amine, among other functional groups known to a person of ordinary skill in the art. Suitable carbonyl functional groups can include carboxylic acid, ester, amide, or ketones.

The monodentate ligand can comprise a single carboxylic acid functional group. Suitable monodentate ligands comprising carboxylic acid can include compounds with the formula R-COOH, wherein R is any organic structure. Suitable monodentate ligands comprising carboxylic acid can also include aliphatic carboxylic acid, aromatic carboxylic acid, sugar acid, salts thereof, and/or combinations thereof.

The aliphatic carboxylic acid can comprise a carboxylic acid functional group attached to a linear hydrocarbon chain, a branched hydrocarbon chain, and/or cyclic hydrocarbon molecule. The aliphatic carboxylic acid can be fully saturated or unsaturated and have one or more alkene and/or alkyne functional groups. Other functional groups can be present and bonded to the hydrocarbon chain, including halogenated variants of the hydrocarbon chain. The aliphatic carboxylic acid can also include hydroxyl acids, which are organic compounds with an alcohol functional group in the alpha, beta, or gamma position relative to the carboxylic acid functional group. A suitable alpha hydroxy acid includes lactic acid and/or a salt thereof.

The aromatic carboxylic acid can comprise a carboxylic acid functional group attached to at least one aromatic functional group. Suitable aromatic carboxylic acid groups can include benzoic acid, salicylic acid, and/or combinations thereof.

The carboxylic acid can include formic acid, acetic acid, propionic acid, butyric acid, valeric acid, caproic acid, enanthic acid, caprylic acid, ascorbic acid, benzoic acid, caprylic acid, cholic acid, glycine, alanine, valine, isoleucine, leucine, phenylalanine, linoleic acid, niacin, oleic acid, propanoic acid, sorbic acid, stearic acid, gluconate, lactate, carbonate, chloroacetic acid, dichloroacetic acid, trichloroacetic acid, salts thereof, and/or combinations thereof.

The oral care composition can include from about 0.01% to about 10%, from about 0.1% to about 15%, from about 1% to about 5%, or from about 0.0001 to about 25%, by weight of the composition, of the monodentate ligand.

### Polydentate Ligand

The oral care composition can comprise polydentate ligand having a molecular weight (MW) of less than 1000 g/mol or less than 2500 g/mol. A polydentate ligand has at least two functional groups that can interact with the central atom, such as a tin ion. Additionally, the polydentate ligand must be suitable for the use in oral care composition, which can be include being listed in Generally Regarded as Safe (GRAS) list with the United States Food and Drug Administration or another suitable list in a jurisdiction of interest.

The polydentate ligand, as described herein, can include at least two functional groups that can chelate to, associate with, and/or bond to tin. The polydentate ligand can comprise a bidentate ligand (i.e., with two functional groups), tridentate (i.e., with three functional groups), tetradentate (i.e., with four functional groups), etc.

Suitable functional groups that can chelate to, associate with, and/or bond to tin include carbonyl, phosphate, nitrate, amine, among other functional groups known to a person of ordinary skill in the art. Suitable carbonyl functional groups can include carboxylic acid, ester, amide, or ketones.

The polydentate ligand can comprise two or more carboxylic acid functional groups. Suitable polydentate ligands comprising carboxylic acid can include compounds with the formula HOOC-R-COOH, wherein R is any organic structure. Suitable polydentate ligands comprising two or more carboxylic acid can also include dicarboxylic acid, tricarboxylic acid, tetracarboxylic acid, *etc*.

Other suitable polydentate ligands include compounds comprising at least two phosphate functional groups. Thus, the polydentate ligand can comprise polyphosphate, as described herein.

Other suitable polydentate ligands include hops beta acids, such as lupulone, colupulone, adlupulone, and/or combinations thereof. The hops beta acid can be synthetically derived and/or extracted from a natural source.

The polydentate ligand can also include phosphate as the functional group to interact with the tin. Suitable phosphate compounds include phosphate salts, organophosphates, or combinations thereof. Suitable phosphate salts include salts of orthophosphate, hydrogen phosphate, dihydrogen phosphate, alkylated phosphates, and combinations thereof. The polydentate ligand can comprise oxalic acid, malonic acid, succinic acid, glutaric acid, adipic acid, pimelic acid, suberic acid, azerlaic acid, sebacic acid, undecanedioic acid, dodecanedioic acid, brassylic acid, thapsic acid, japanic acid, phellogenic acid, equisetolic acid, maleic acid, malic acid, tartaric acid, phthalic acid, citric acid, phytic acid, pyrophosphate, tripolyphosphate, tetrapolyphosphate, hexametaphosphate, salts thereof, and/or combinations thereof.

The oral care composition can include from about 0.01% to about 10%, from about 0.1% to about 15%, from about 1% to about 5%, or from about 0.0001 to about 25%, by weight of the composition, of the polydentate ligand.

### Thickening Agent

The oral care composition can comprise one or more thickening agents. Thickening agents can be useful in the oral care compositions to provide a gelatinous structure that stabilizes the composition against phase separation. Suitable thickening agents include polysaccharides, polymers, and/or silica thickeners.

The thickening agent can comprise one or more polysaccharides. Some non-limiting examples of polysaccharides include starch; glycerite of starch; gums such as gum karaya (sterculia gum), gum tragacanth, gum arabic, gum ghatti, gum acacia, xanthan gum, guar gum and cellulose gum; magnesium aluminum silicate (Veegum); carrageenan; sodium alginate; agar-agar; pectin; gelatin; cellulose compounds such as cellulose, microcrystalline cellulose, carboxymethyl cellulose, hydroxyethyl cellulose, hydroxypropyl cellulose, hydroxymethyl cellulose, hydroxymethyl carboxypropyl cellulose, methyl cellulose, ethyl cellulose, and sulfated cellulose; natural and synthetic clays such as hectorite clays; and mixtures thereof.

Other polysaccharides that are suitable for use herein include carageenans, gellan gum, locust bean gum, xanthan gum, carbomers, poloxamers, modified cellulose, and mixtures thereof. Carageenan is a polysaccharide derived from seaweed. There are several types of carageenan that may be distinguished by their seaweed source and/or by their degree of and position of sulfation. The thickening agent can comprise kappa carageenans, modified kappa carageenans, iota carageenans, modified iota carageenans, lambda carrageenan, and mixtures thereof. Carageenans suitable for use herein include those commercially available from the FMC Company under the series designation "Viscarin," including but not limited to Viscarin TP 329, Viscarin TP 388, and Viscarin TP 389.

The thickening agent can comprise one or more polymers. The polymer can be a polyethylene glycol (PEG), a polyvinylpyrrolidone (PVP), polyacrylic acid, a polymer derived from at least one acrylic acid monomer, a copolymer of maleic anhydride and methyl vinyl ether, a crosslinked polyacrylic acid polymer, of various weight percentages of the oral care composition as well as various ranges of average molecular ranges. Alternatively, the oral care composition can be free of, essentially free of, or substantially free of a copolymer of maleic anhydride and methyl vinyl ether. The polymer can comprise polyacrylate crosspolymer, such as polyacrylate crosspolymer-6. Suitable sources of polyacrylate crosspolymer-6 can include Sepimax Zen^{TM} commercially available from Seppic.

The thickening agent can comprise inorganic thickening agents. Some non-limiting examples of suitable inorganic thickening agents include colloidal magnesium aluminum silicate, silica thickeners. Useful silica thickeners include, for example, include, as a non-limiting example, an amorphous precipitated silica such as ZEODENT^{®} 165 silica. Other non-limiting silica thickeners include ZEODENT^{®} 153, 163, and 167, and ZEOFREE^{®} 177 and 265 silica products, all available from Evonik Corporation, and AEROSIL^{®} fumed silicas.

The oral care composition can comprise from 0.01% to about 15%, from 0.1% to about 10%, from about 0.2% to about 5%, or from about 0.5 % to about 2% of one or more thickening agents.

### Abrasive

The oral care composition of embodiments of the present invention can comprise an abrasive. Abrasives can be added to oral care formulations to help remove surface stains from teeth. The oral care can include a calcium abrasive and/or a non-calcium abrasive, such as a silica abrasive.

The oral care composition can comprise a calcium abrasive. The calcium abrasive can be any suitable abrasive compound that can provide calcium ions in an oral care composition and/or deliver calcium ions to the oral cavity when the oral care composition is applied to the oral cavity. The oral care composition can comprise from about 5% to about 70%, from about 10% to about 60%, from about 20% to about 50%, from about 25% to about 40%, or from about 1% to about 50% of a calcium abrasive. The calcium abrasive can comprise one or more calcium abrasive compounds, such as calcium carbonate, precipitated calcium carbonate (PCC), ground calcium carbonate (GCC), chalk, dicalcium phosphate, calcium pyrophosphate, and/or mixtures thereof.

The oral care composition can comprise a non-calcium abrasive such as bentonite, silica gel (by itself, and of any structure), precipitated silica, amorphous precipitated silica (by itself, and of any structure as well), hydrated silica, perlite, titanium dioxide, calcium pyrophosphate, dicalcium phosphate dihydrate, alumina, hydrated alumina, calcined alumina, aluminum silicate, insoluble sodium metaphosphate, insoluble potassium metaphosphate, insoluble magnesium carbonate, zirconium silicate, particulate thermosetting resins and other suitable abrasive materials. Such materials can be introduced into the oral care compositions to tailor the polishing characteristics of the target dentifrice formulation. The oral care composition can comprise from about 5% to about 70%, from about 10% to about 50%, from about 10% to about 60%, from about 20% to about 50%, from about 25% to about 40%, or from about 1% to about 50%, by weight of the oral care composition, of the non-calcium abrasive.

Alternatively, the oral care composition can be essentially free of, substantially free of, essentially free of, or free of silica, alumina, or any other non-calcium abrasive. The oral care composition can comprise less than about 5%, less than about 1%, less than about 0.5%, less than about 0.1%, or 0% of a non-calcium abrasive, such as silica and/or alumina.

The oral care composition can also comprise a silica abrasive, such as silica gel (by itself, and of any structure), precipitated silica, amorphous precipitated silica (by itself, and of any structure as well), hydrated silica, and/or combinations thereof. The oral care composition can comprise from about 5% to about 70%, from about 10% to about 60%, from about 10% to about 50%, from about 20% to about 50%, from about 25% to about 40%, or from about 1% to about 50% of a silica abrasive.

Where the oral care composition comprises a dicarboxylic acid, the oral care composition can include a low level of or no abrasive as the dicarboxylic acid can provide a high enough whitening benefit that an abrasive is not necessary.

While mouth rinse compositions typically do not include abrasive, dentifrice compositions typically do include abrasive. However, the dentifrice compositions and/or toothpaste compositions of embodiments of the present invention can include a low level of or no abrasive. As such, the oral care composition or dentifrice composition can comprise less than about 5%, from about 0.5% to about 2%, or less than about 2%, by weight of the composition, of abrasive. The oral care composition or dentifrice composition can also be essentially free of, substantially free of, or free of abrasive.

### Prenylated Flavonoids

The oral care composition can comprise prenylated flavonoid. Flavonoids are a group of natural substances found in a wide range of fruits, vegetables, grains, bark, roots, stems, flowers, tea, and wine. Flavonoids can have a variety of beneficial effects on health, such as antioxidative, anti-inflammatory, antimutagenic, anticarcinogenic, and antibacterial benefits. Prenylated flavonoids are flavonoids that include at least one prenyl functional group (3-methylbut-2-en-1-yl, as shown in Formula IX), which has been previously identified to facilitate attachment to cell membranes. Thus, while not wishing to being bound by theory, it is believed that the addition of a prenyl group, i.e. prenylation, to a flavonoid can increase the activity of the original flavonoid by increasing the lipophilicity of the parent molecule and improving the penetration of the prenylated molecule into the bacterial cell membrane. Increasing the lipophilicity to increase penetration into the cell membrane can be a double-edged sword because the prenylated flavonoid will tend towards insolubility at high Log P values (high lipophilicity). Log P can be an important indicator of antibacterial efficacy.

As such, the term prenylated flavonoids can include flavonoids found naturally with one or more prenyl functional groups, flavonoids with a synthetically added prenyl functional group, and/or prenylated flavonoids with additional prenyl functional groups synthetically added.

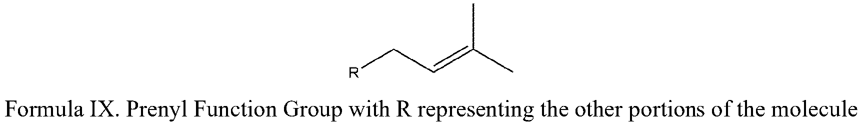

Other suitable functionalities of the parent molecule that improve the structure-activity relationship (e.g,. structure-MIC relationship) of the prenylated molecule include additional heterocycles containing nitrogen or oxygen, alkylamino chains, or alkyl chains substituted onto one or more of the aromatic rings of the parent flavonoid.

Flavonoids can have a 15-carbon skeleton with at least two phenyl rings and at least one heterocyclic ring. Some suitable flavonoid backbones can be shown in Formula X (flavone backbone), Formula XI (isoflavan backbone), and/or Formula XII (neoflavonoid backbone).

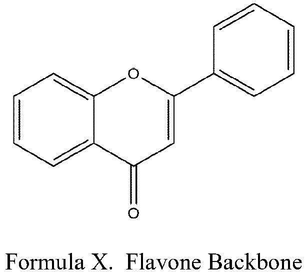

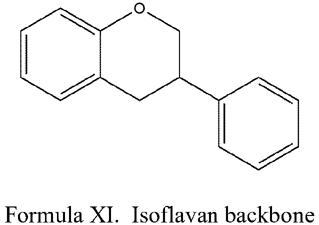

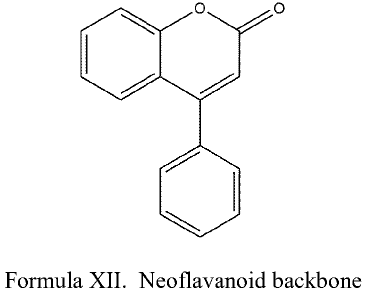

Other suitable subgroups of flavonoids include anthocyanidins, anthoxanthins, flavanones, flavanonols, flavans, isoflavonoids, chalcones and/or combinations thereof.

Prenylated flavonoids can include naturally isolated prenylated flavonoids or naturally isolated flavonoids that are synthetically altered to add one or more prenyl functional groups through a variety of synthetic processes that would be known to a person of ordinary skill in the art of synthetic organic chemistry.

Other suitable prenylated flavonoids can include Bavachalcone, Bavachin, Bavachinin, Corylifol A, Epimedin A, Epimedin A1, Epimedin B, Epimedin C, Icariin, Icariside I, Icariside II, Icaritin, Isobavachalcone, Isoxanthohumol, Neobavaisoflavone, 6-Prenylnaringenin, 8-Prenylnaringenin, Sophoraflavanone G, (-)-Sophoranone, Xanthohumol, Quercetin, Macelignan, Kuraridin, Kurarinone, Kuwanon G, Kuwanon C, Panduratin A, 6-geranylnaringenin, Australone A, 6,8-Diprenyleriodictyol, dorsmanin C, dorsmanin F, 8-Prenylkaempferol, 7-O-Methylluteone, luteone, 6-prenylgenistein, isowighteone, lupiwighteone, and/or combinations thereof. Other suitable prenylated flavonoids include cannflavins, such as Cannflavin A, Cannflavin B, and/or Cannflavin C.

Preferably, the prenylated flavonoid has a high probability of having a MIC of less than about 25 ppm for *S. aureus*, a gram-positive bacterium. Suitable prenylated flavonoids include Bavachin, Bavachinin, Corylifol A, Icaritin, Isoxanthohumol, Neobavaisoflavone, 6-Prenylnaringenin, 8-Prenylnaringenin, Sophoraflavanone G, (-)-Sophoranone, Kurarinone, Kuwanon C, Panduratin A, and/or combinations thereof.

Preferably, the prenylated flavonoid has a high probability of having a MIC of less than about 25 ppm for *E. coli*, a gram-negative bacterium. Suitable prenylated flavonoids include Bavachinin, Isoxanthohumol, 8-Prenylnaringenin, Sophoraflavanone G, Kurarinone, Panduratin A, and/or combinations thereof.

Approximately 1000 prenylated flavonoids have been identified from plants. According to the number of prenylated flavonoids reported before, prenylated flavonones are the most common subclass and prenylated flavanols is the rarest sub-class. Even though natural prenylated flavonoids have been detected to have diversely structural characteristics, they have a narrow distribution in plants, which are different to the parent flavonoids as they are present almost in all plants. Most of prenylated flavonoids are found in the following families, including *Cannabaceae*, *Guttiferae*, *Leguminosae*, *Moraceae*, *Rutaceae* and *Umbelliferae*. *Leguminosae* and *Moraceae*, due to their consumption as fruits and vegetables, are the most frequently investigated families and many novel prenylated flavonoids have been explored. *Humulus lupulus* of the *Cannabaceae* include 8-prenylnaringenin and xanthohumol, which can play a role in the health benefits of beer.

The prenylated flavonoid can be incorporated through a hops extract, incorporated in a separately added extract, or added as a separate component of the oral care compositions disclosed herein.

Suitable prenylated flavonoids can have a particular octanol-water partitioning coefficient. The octanol-water partitioning coefficient can be used to predict the lipophilicity of a compound. Without wishing to being bound by theory, it is believed that compounds that fall within the ranges described herein will be able to enter and/or disrupt the primarily hydrophobic phospholipid bilayer that makes of the cell membrane of microorganisms. Thus, the octanol-water partitioning coefficient can be correlated to the antibacterial effect of prenylated flavonoids. Suitable prenylated flavonoids can have a log P of at least about 2, at least about 4, from about 2 to about 10, from about 4 to about 10, from about 4 to about 7, or from about 4 to about 7.

The oral care composition can comprise at least about 0.001%, from about 0.001% to about 5%, from about 0.01% to about 2%, from about 0.0001% to about 2%, or at least about 0.05% of prenylated flavonoid.

### Amino Acid

The oral care composition can comprise amino acid. The amino acid can comprise one or more amino acids, peptide, and/or polypeptide, as described herein.

Amino acids, as in Formula XIII, are organic compounds that contain an amine functional group, a carboxyl functional group, and a side chain (R in Formula XIII) specific to each amino acid. Suitable amino acids include, for example, amino acids with a positive or negative side chain, amino acids with an acidic or basic side chain, amino acids with polar uncharged side chains, amino acids with hydrophobic side chains, and/or combinations thereof. Suitable amino acids also include, for example, arginine, histidine, lysine, aspartic acid, glutamic acid, serine, threonine, asparagine, glutamine, cysteine, selenocysteine, glycine, proline, alanine, valine, isoleucine, leucine, methionine, phenylalanine, tyrosine, tryptophan, citrulline, ornithine, creatine, diaminobutanoic acid, diaminoproprionic acid, salts thereof, and/or combinations thereof.

Suitable amino acids include the compounds described by Formula XIII, either naturally occurring or synthetically derived. The amino acid can be zwitterionic, neutral, positively charged, or negatively charged based on the R group and the environment. The charge of the amino acid, and whether particular functional groups, can interact with tin at particular pH conditions, would be well known to one of ordinary skill in the art.

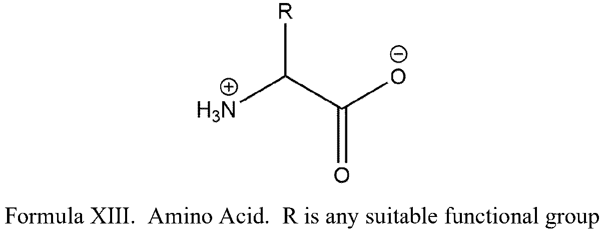

Suitable amino acids include one or more basic amino acids, one or more acidic amino acids, one or more neutral amino acids, or combinations thereof.

The oral care composition can comprise from about 0.01% to about 20%, from about 0.1% to about 10%, from about 0.5% to about 6%, or from about 1% to about 10 % of amino acid, by weight of the oral care composition.

The term "neutral amino acids" as used herein include not only naturally occurring neutral amino acids, such as alanine, asparagine, cysteine, glutamine, glycine, isoleucine, leucine, methionine, phenylalanine, proline, serine, threonine, tryptophan, tyrosine, valine, but also biologically acceptable amino acids which have an isoelectric point in range of pH 5.0 to 7.0. The biologically preferred acceptable neutral amino acid has a single amino group and carboxyl group in the molecule or a functional derivative hereof, such as functional derivatives having an altered side chain albeit similar or substantially similar physio chemical properties. In a further embodiment the amino acid would be at minimum partially water soluble and provide a pH of less than 7 in an aqueous solution of 1g/1000ml at 25 °C.

Accordingly, neutral amino acids suitable for use in embodiments of the present invention include, but are not limited to, alanine, aminobutyrate, asparagine, cysteine, cystine, glutamine, glycine, hydroxyproline, isoleucine, leucine, methionine, phenylalanine, proline, serine, taurine, threonine, tryptophan, tyrosine, valine, salts thereof, or mixtures thereof. Preferably, the neutral amino acids used in embodiments of the present invention may include asparagine, glutamine, glycine, salts thereof, or mixtures thereof. The neutral amino acids may have an isoelectric point of 5.0, or 5.1, or 5.2, or 5.3, or 5.4, or 5.5, or 5.6, or 5.7, or 5.8, or 5.9, or 6.0, or 6.1, or 6.2, or 6.3, or 6.4, or 6.5, or 6.6, or 6.7, or 6.8, or 6.9, or 7.0, in an aqueous solution at 25 °C. Preferably, the neutral amino acid is selected from proline, glutamine, or glycine, more preferably in its free form (i.e., uncomplexed). If the neutral amino acid is in its salt form, suitable salts include salts known in the art to be pharmaceutically acceptable salts considered to be physiologically acceptable in the amounts and concentrations provided. Preferably the neutral amino acid is present in the amount of from about 0.0001% to about 10%, preferably from about 0.05% to about 5%, preferably from about 0.1% to about 3%, preferably from about 0.5% to about 3%, preferably from about 1% to about 3%, by weight of the composition. In one aspect, the neutral amino acid is glutamine (or salt thereof). In another aspect, the neutral amino acid is proline (or salt thereof). In yet another aspect, the neutral amino acid is glycine (or salt thereof).

The oral care composition can comprise from about 0.0001% to about 20%, from about 0.1% to about 10%, from about 0.5% to about 6%, or from about 1% to about 10 % of neutral amino acid, by weight of the oral care composition.

### Whitening Agent

The oral care composition may comprise from about 0.1% to about 10%, from about 0.2% to about 5%, from about 1% to about 5%, or from about 1% to about 15%, by weight of the oral care composition, of a whitening agent. The whitening agent can be a compound suitable for whitening at least one tooth in the oral cavity. The whitening agent may include peroxides, metal chlorites, perborates, percarbonates, peroxyacids, persulfates, dicarboxylic acids, and combinations thereof. Suitable peroxides include solid peroxides, hydrogen peroxide, urea peroxide, calcium peroxide, benzoyl peroxide, sodium peroxide, barium peroxide, inorganic peroxides, hydroperoxides, organic peroxides, and mixtures thereof. Suitable metal chlorites include calcium chlorite, barium chlorite, magnesium chlorite, lithium chlorite, sodium chlorite, and potassium chlorite. Other suitable whitening agents include sodium persulfate, potassium persulfate, peroxydone, 6-phthalimido peroxy hexanoic acid, pthalamidoperoxycaproic acid, or mixtures thereof.

### Humectant

The oral care composition can comprise one or more humectants, have low levels of a humectant, be essentially free of, be substantially free of, or be free of a humectant. Humectants serve to add body or "mouth texture" to an oral care composition or dentifrice as well as preventing the dentifrice from drying out. Suitable humectants include polyethylene glycol (at a variety of different molecular weights), propylene glycol, glycerin (glycerol), erythritol, xylitol, sorbitol, mannitol, butylene glycol, lactitol, hydrogenated starch hydrolysates, and/or mixtures thereof. The oral care composition can comprise one or more humectants each at a level of from 0 to about 70%, from about 5% to about 50%, from about 10% to about 60%, or from about 20% to about 80%, by weight of the oral care composition.

### Water

The oral care composition according to embodiments of the present invention can be anhydrous, a low water formulation, or a high water formulation. In total, the oral care composition can comprise from 0% to about 99%, from about 5% to about 75%, about 20% or greater, about 30% or greater, about 50% or greater, up to about 45%, or up to about 75%, by weight of the composition, of water.

In a high water oral care composition and/or toothpaste formulation, the oral care composition comprises from about 45% to about 75%, by weight of the composition, of water. The high water oral care composition and/or toothpaste formulation can comprise from about 45% to about 65%, from about 45% to about 55%, or from about 46% to about 54%, by weight of the composition, of water. The water may be added to the high water formulation and/or may come into the composition from the inclusion of other ingredients.

In a low water oral care composition and/or toothpaste formulation, the oral care composition comprises from about 5% to about 45%, by weight of the composition, of water. The low water oral care composition can comprise from about 5% to about 35%, from about 10% to about 25%, or from about 20% to about 25%, by weight of the composition, of water. The water may be added to the low water formulation and/or may come into the composition from the inclusion of other ingredients.

In an anhydrous oral care composition and/or toothpaste formulation, the oral care composition comprises less than about 10%, by weight of the composition, of water. The anhydrous composition comprises less than about 5%, less than about 1%, or 0%, by weight of the composition, of water. The water may be added to the anhydrous formulation and/or may come into the composition from the inclusion of other ingredients.

The oral care composition can also be a mouth rinse formulation. A mouth rinse formulation can comprise from about 75% to about 99%, from about 75% to about 95%, or from about 80% to about 95% of water.

The dentifrice composition can also comprise other orally acceptable carrier materials, such as alcohol, humectants, polymers, surfactants, and acceptance improving agents, such as flavoring, sweetening, coloring and/or cooling agents.

### Other Ingredients

The oral care composition can comprise a variety of other ingredients, such as flavoring agents, sweeteners, colorants, preservatives, buffering agents, or other ingredients suitable for use in oral care compositions, as described below.

Flavoring agents also can be added to the oral care composition. Suitable flavoring agents include oil of wintergreen, oil of peppermint, oil of spearmint, clove bud oil, menthol, anethole, methyl salicylate, eucalyptol, cassia, 1-menthyl acetate, sage, eugenol, parsley oil, oxanone, alpha-irisone, marjoram, lemon, orange, propenyl guaethol, cinnamon, vanillin, ethyl vanillin, heliotropine, 4-cis-heptenal, diacetyl, methyl-para-tert-butyl phenyl acetate, and mixtures thereof. Coolants may also be part of the flavor system. Preferred coolants in the present compositions are the paramenthan carboxyamide agents such as N-ethyl-p-menthan-3-carboxamide (known commercially as "WS-3") or N-(Ethoxycarbonylmethyl)-3-p-menthanecarboxamide (known commercially as "WS-5"), and mixtures thereof. A flavor system is generally used in the compositions at levels of from about 0.001 % to about 5%, by weight of the oral care composition. These flavoring agents generally comprise mixtures of aldehydes, ketones, esters, phenols, acids, and aliphatic, aromatic and other alcohols.

Sweeteners can be added to the oral care composition to impart a pleasing taste to the product. Suitable sweeteners include saccharin (as sodium, potassium or calcium saccharin), cyclamate (as a sodium, potassium or calcium salt), acesulfame-K, thaumatin, neohesperidin dihydrochalcone, ammoniated glycyrrhizin, dextrose, levulose, sucrose, mannose, sucralose, stevia, and glucose.

Colorants can be added to improve the aesthetic appearance of the product. Suitable colorants include without limitation those colorants approved by appropriate regulatory bodies such as the FDA and those listed in the European Food and Pharmaceutical Directives and include pigments, such as TiO₂, and colors such as FD&C and D&C dyes.

Preservatives also can be added to the oral care compositions to prevent bacterial growth. Suitable preservatives approved for use in oral compositions such as methylparaben, propylparaben, benzoic acid, and sodium benzoate can be added in safe and effective amounts.

Titanium dioxide may also be added to the present composition. Titanium dioxide is a white powder which adds opacity to the compositions. Titanium dioxide generally comprises from about 0.25% to about 5%, by weight of the oral care composition.

Other ingredients can be used in the oral care composition, such as desensitizing agents, healing agents, other caries preventative agents, chelating/sequestering agents, vitamins, amino acids, proteins, other anti-plaque/anti-calculus agents, opacifiers, antibiotics, anti-enzymes, enzymes, pH control agents, oxidizing agents, antioxidants, and the like.

### Oral Care Composition Forms

Suitable compositions forms include emulsion compositions, such as the emulsions compositions of U.S. Patent No. 11,147,753, which is herein incorporated by reference in its entirety, unit-dose compositions, such as the unit-dose compositions of U.S. Patent Application Publication No. 2019/0343732, which is herein incorporated by reference in its entirety, leave-on oral care compositions, jammed emulsions, such as the jammed oil-in-water emulsions of U.S. Patent No. 11,096,874, which is herein incorporated by reference in its entirety, dentifrice compositions, mouth rinse compositions, mouthwash compositions, tooth gel, subgingival gel, mouth rinse, mousse, foam, mouth spray, lozenge, chewable tablet, chewing gum, tooth whitening strips, floss and floss coatings, breath freshening dissolvable strips, denture care products, denture adhesive products, or combinations thereof.

### Methods

The oral care compositions, as described herein, can lead to oral health benefits, such as the treatment, reduction, and/or prevention of caries, cavities, gingivitis, and/or combinations thereof and/or the whitening of teeth, removing stain from teeth, and/or preventing the accumulation of stain from teeth when applied to the oral cavity. For example, a user can dispense at least a one-inch strip of a suitable oral care composition, as described herein, onto an oral care implement, such as a toothbrush, applicator, and/or tray, and applied to the oral cavity and/or teeth.

The user can be instructed to brush teeth thoroughly for at least 30 seconds, at least one minute, at least 90 seconds, or at least two minutes at least once, at least twice, or at least three times per day. The user can also be instructed to expectorate the oral care composition after the completion of the brush procedure.

The user can also be instructed to rinse with a mouthwash and/or mouth rinse composition after the completion of the brush procedure or instead of the brush procedure. The user can be instructed to swish the oral care composition thoroughly for at least 30 seconds, at least one minute, at least 90 seconds, or at least two minutes at least once, at least twice, or at least three times per day. The user can also be instructed to expectorate the oral care composition after the completion of the procedure.

The oral care compositions according to embodiments of the present invention can be used in the treatment, reduction, and/or prevention of caries, cavities, gingivitis, and/or combinations thereof. The oral care compositions according to embodiments of the present invention can be used to provide a whitening benefit, such as the whitening of teeth, removing stain from teeth, and/or preventing the accumulation of stain from teeth. For example, as described herein, hops beta acid can be useful as an antigingivitis agent. Thus, the addition of hops to any oral care composition can provide antigingivitis protection.

The oral care composition can include primary packaging, such as a tube, bottle, and/or tub. The primary package can be placed within secondary package, such as a carton, shrink wrap, or the like. Instructions for use of the oral care composition can be printed on the primary package and/or the secondary package. The scope of the method is intended to include instructions provided by a manufacturer, distributor, and/or producer of the oral care composition.

If the oral care composition is a toothpaste, the user can be instructed to dispense the toothpaste from the toothpaste tube.

The user can be instructed to apply a portion of the toothpaste onto a toothbrush. The portion of the toothpaste can be of any suitable shape, such as strip, a pea-sized amount, or various other shapes that would fit onto any mechanical and/or manual brush head. The user can be instructed to apply a strip of the toothpaste that is at least about 1 inch, at least about 0.5 inch, at least 1 inch, and/or at least 0.5 inch long to the bristles of a toothbrush, such as soft-bristled toothbrush.

The user can be instructed to apply pea-sized or grain of rice-sized portion of the toothpaste to the bristles of a toothbrush, such as in the case of use by children of less than 6 years old and/or less than 2 years old.

The user can be instructed to brush their teeth for at least about 30 seconds, at least about 1 minute, at least about 90 seconds, at least about 2 minutes, at least 30 seconds, at least 1 minute, at least 90 seconds, and/or at least 2 minutes.

The user can be instructed to brush their teeth thoroughly and/or as directed by a physician and/or dentist.

The user can be instructed to brush their teeth after each meal. The user can be instructed to brush their teeth at least once per day, at least twice per day, and/or at least three times per day. The user can be instructed to brush their teeth no more than three times a day, such as to prevent Sn staining. The user can be instructed to brush their teeth in the morning and/or in the evening prior to sleeping.

The user can be instructed to not swallow the toothpaste composition due to the inclusion of ingredients that are not suitable for ingestion, such as fluoride. However, in the case of an oral care composition comprising hops, but free of fluoride, the user may not need to be instructed to not swallow the toothpaste. The user may be instructed to expectorate (or spit out) the toothpaste composition after the cessation of the brushing cycle.

If the oral care composition is a mouth rinse, the user can be instructed to dispense the mouth rinse from a bottle containing the mouth rinse.

The user can be instructed to use the mouth rinse at least once a day, at least twice a day, and/or at least three times a day.

The user can be instructed to use the mouth rinse composition after the use of toothpaste and/or floss.

The user can be instructed to swish a portion of rinse in the oral cavity, such as between the teeth, for a period of time. The user can be instructed to vigorously swish a portion of the rinse.

The user can be instructed to use be from about 5 mL to about 50 mL, from about 10 mL to about 40 mL, 10 mL, 20 mL, 25 mL, 30 mL, 40 mL, 2 teaspoonfuls, and/or 4 teaspoonfuls of mouth rinse.

The user can be instructed to swish the mouth rinse for at least about 30 seconds, at least about 1 minute, at least about 90 seconds, at least about 2 minutes, at least 30 seconds, at least 1 minute, at least 90 seconds, and/or at least 2 minutes.

The user can be instructed to not swallow the mouth rinse composition due to the inclusion of ingredients that are not suitable for ingestion, such as fluoride. However, in the case of an oral care composition comprising hops, but free of fluoride, the user may not need to be instructed to not swallow the mouth rinse. The user may be instructed to expectorate (or spit out) the mouth rinse composition after the cessation of the rinse cycle.

The usage instructions for the oral care composition, such as for a toothpaste composition and/or a mouth rinse composition, can vary based on age. For example, adults and children that are at least 6 or at least 2 can have one usage instruction while children under 6 or under 2 can have a second usage instruction.

The oral care composition comprising hops, as described herein, can be useful as medicament, such as in an anticavity and/or antigingivitis treatment, as described herein. Suitable medicaments include oral care compositions, toothpaste compositions, mouth rinse compositions, floss coatings, chewing gums, and/or other suitable compositions to be applied in the oral cavity.

Additionally, the oral care composition, as described herein, can be used to reduce the number and/or intensity of white spots on teeth, which can be attributable to caries presence within the oral cavity. Or the oral care composition, as described herein, can be used to reduce the redness, puffiness, tenderness, and/or swollenness of gums at the gumline immediately adjacent the surfaces of the teeth, which can be attributable to gingivitis presence within the oral cavity.

### COMBINATIONS

A. An oral care composition comprising:
   hops; and
   one or more surfactants,
   wherein a solution comprising the hops and the one or more surfactants is clear or slightly hazy in a pH range of about 4.5 to about 9.
B. The oral care composition of A, wherein if the one or more surfactants comprise sodium cocoyl glutamate, lauryl glucoside, or poloxamer, then the oral care composition comprises at least two surfactants.
C. The oral care composition of A or B, wherein the one or more surfactants consists of a single surfactant, and the single surfactant does not comprise sodium cocoyl glutamate, lauryl glucoside, or poloxamer.
D. The oral care composition of any one of A-C, wherein the one or more surfactants are selected from the group consisting of sodium lauryl sulfate, cocamidopropyl betaine, sodium cocoyl glutamate, sodium methyl cocoyl taurate, or a combination thereof.
E. The oral care composition of any one of A-C, wherein the one or more surfactants consist essentially of sodium lauryl sulfate, cocamidopropyl betaine, sodium methyl cocoyl taurate, or a combination thereof.
F. The oral care composition of any one of A-E, wherein the oral care composition comprises from greater than 0% to about 5%, preferably greater than 0% to about 2%, by weight of the oral care composition, of the sodium lauryl sulfate, and the pH range is about 7 to about 9.
G. The oral care composition of any one of A-F, wherein the oral care composition comprises about 2%, by weight of the oral care composition, of the sodium lauryl sulfate, and the pH range is about 7 to about 9.
H. The oral care composition of any one of A-G, wherein the oral care composition comprises from greater than 0% to about 2%, by weight of the oral care composition, of the cocamidopropyl betaine.
I. The oral care composition of any one of A-H, wherein the oral care composition comprises from greater than 0% to about 5%, preferably greater than 0% to about 1%, by weight of the oral care composition, of the cocamidopropyl betaine, and the pH range is about 5 to about 9.
J. The oral care composition of any one of A-I, wherein the oral care composition comprises from greater than 0% to about 5%, preferably about 1% to about 2%, by weight of the oral care composition, of the cocamidopropyl betaine.
K. The oral care composition of any one of A-J, wherein the oral care composition comprises from greater than 0% to about 5%, preferably greater than 0% to less than 2%, by weight of the oral care composition, of the sodium methyl cocoyl taurate.
L. The oral care composition of any one of A-K, wherein the one or more surfactants comprises sodium lauryl sulfate as a main surfactant and a co-surfactant comprising cocamidopropyl betaine, sodium methyl cocoyl taurate, lauryl glucoside, or poloxamer.
M. The oral care composition of any one of A-L, wherein the one or more surfactants comprises cocamidopropyl betaine as a main surfactant and a co-surfactant comprising sodium lauryl sulfate, sodium methyl cocoyl taurate, lauryl glucoside, or poloxamer.
N. The oral care composition of M, wherein the co-surfactant comprises sodium lauryl sulfate, preferably wherein the pH is from about 4.5 to about 8
O. The oral care composition of N, wherein the co-surfactant comprises poloxamer, preferably wherein the pH is from about 7 to about 8.
P. The oral care composition of any one of A-L, wherein the one or more surfactants comprises sodium methyl cocoyl taurate as a main surfactant and a co-surfactant comprising sodium lauryl sulfate, cocamidopropyl betaine, or lauryl glucoside.
Q. The oral care composition of any one of A-L, wherein the one or more surfactants comprises lauryl glucoside as a main surfactant and a co-surfactant comprising sodium lauryl sulfate, preferably wherein the pH is from about 8 to about 9.
R. The oral care composition of any one of A-L, wherein the one or more surfactants comprises poloxamer as a main surfactant and a co-surfactant comprising sodium lauryl sulfate, cocamidopropyl betaine, sodium cocoyl glutamate, sodium methyl cocoyl taurate, or lauryl glucoside.
S. The oral care composition of R, wherein the co-surfactant comprises LG, preferably wherein the pH is from about 8 to about 9.
T. The oral care composition of any one of A-S, further comprising an abrasive.
U. The oral care composition of T, wherein the abrasive comprises calcium abrasive.
V. The oral care composition of T, wherein the calcium abrasive comprises calcium carbonate.
W. The oral care composition of any one of A-V, wherein the oral care composition is free of silica.
X. The oral care composition of any one of A-V, wherein the oral care composition is free of fluoride.
Y. The oral care composition of any one of A-V, further comprising from about 0.01% to about 10%, by weight of the composition, of a metal ion source.
Z. The oral care composition of Y, wherein the metal ion source comprises tin, zinc, or combinations thereof.
AA. The oral care composition of any one of A-Z, wherein the hops beta acid comprises only non-hydrogenated hops beta acid.
BB. The oral care composition of any one of A-AA, wherein the oral care composition comprises from greater than 0% to about 5%, preferably greater than 0% to about 3%, more preferably greater than 0% to about 2%, by weight of the composition, of the one or more surfactants.
CC. An oral care composition comprising:
   hops acid;
   flavor; and
   one or more surfactants,
   wherein the oral care composition has an added salt concentration of at least 2.5%, by weight of the oral care composition, and a pH of about 5 to about 7.5, and
   wherein the oral care composition has phase stability.
DD. The oral care composition of CC, wherein the one or more surfactants comprises sodium lauryl sulfate.
EE. The oral care composition of DD, wherein the one or more surfactants further comprises polysorbate 80, lauryl glucoside, sodium methyl cocoyl taurate, sodium cocoyl glutamate, or a combination thereof.
FF. The oral care composition of CC or DD, wherein the one or more surfactants is free of poloxamer, polysorbate 80, or a combination thereof.
GG. The oral care composition of any one of CC-FF, wherein the added salt concentration is at least 3%.
HH. The oral care composition of any one of claims CC-GG, wherein a weight percent ratio of added salt to water is 0.05 or greater, or preferably about 0.1 or greater.
II. The oral care composition of any one of claims CC-HH, wherein the composition is free of zinc lactate.
JJ. The oral care composition of any one of claims CC-II, wherein the composition comprises sodium citrate.
KK. The oral care composition of any one of claims CC-JJ, wherein the composition is a dentifrice composition.
LL. The oral care composition of any one of claims CC-KK, wherein the flavor comprises a mint flavor, a wintergreen flavor, or a combination thereof.

### EXAMPLES

The invention is further illustrated by the following examples, which are not to be construed in any way as imposing limitations to the scope of this invention. Various other aspects, modifications, and equivalents thereof which, after reading the description herein, may suggest themselves to one of ordinary skill in the art without departing from the spirit of the present invention or the scope of the appended claims.

### Evaluation of Hops Stability in Rinse Compositions

TABLE 1 and TABLE 2 show compositions with hops from a hops beta acid extract (Hops) and optionally one or more surfactants including: sodium lauryl sulfate (SLS), cocamidopropyl betaine (CAPB), sodium cocoyl glutamate (SCG), sodium methyl cocoyl taurate (SMCT), lauryl glucoside (LG), and poloxamer (POL). The compositions in TABLE 1 include a single surfactant at different concentrations, and the compositions in TABLE 2 include a main surfactant at 1% (neat) with a co-surfactant at 0.33% (neat).

The hops compositions of TABLE 1 and TABLE 2 were made by first making a 1.0 M NaCl stock solution (60 g NaCl in 1 L of water). The pH of the NaCl stock solution was adjusted to pH 9 with 1 M NaOH. Surfactant was then added to a 100 mL volumetric flask according to the examples in TABLE 1 and TABLE 2. An amount of NaCl stock solution was added to bring the volume to 100 mL. Two, 50 mL portions of the dissolve surfactant/NaCl solution were drawn into two 60 mL Luer lock syringes. Two, 0.25 g portions of a Hops Beta Acid Extract solution, as described in TABLE 3, were added into two additional 60 mL Luer lock syringes. Excess air was removed from all syringes. The syringes containing the NaCl and optional surfactant solution and the corresponding syringes containing the Hops Beta Acid Extract were coupled together using a Luer lock coupler. The syringe pistons were depressed rapidly to push the fluid from one syringe to the other syringe. The process was repeated 20 times until the fluids were mixed. A cross-shaped stir bar was added to a 150 mL beaker. The syringes were decoupled and the contents from both syringes were added to the 150 mL beaker. In this manner, each example from TABLE 1 and TABLE 2 was made and assessed for hops solubility. The pH of the resulting solutions was about 9 in each case.

**TABLE 1. Hops Compositions**

| Example | SLS (wt%) | CAPB (wt%) | SCG (wt %) | SMCT (wt %) | LG (wt %) | POL (wt %) | Hops (wt %) |
|---|---|---|---|---|---|---|---|
| 1 | 0 | 0 | 0 | 0 | 0 | 0 | 0.225 |
| 2 | 0.5 | 0 | 0 | 0 | 0 | 0 | 0.225 |
| 3 | 1.0 | 0 | 0 | 0 | 0 | 0 | 0.225 |
| 4 | 2.0 | 0 | 0 | 0 | 0 | 0 | 0.225 |
| 5 | 0 | 0.5 | 0 | 0 | 0 | 0 | 0.225 |
| 6 | 0 | 1.0 | 0 | 0 | 0 | 0 | 0.225 |
| 7 | 0 | 2.0 | 0 | 0 | 0 | 0 | 0.225 |
| 8 | 0 | 0 | 0.5 | 0 | 0 | 0 | 0.225 |
| 9 | 0 | 0 | 1.0 | 0 | 0 | 0 | 0.225 |
| 10 | 0 | 0 | 2.0 | 0 | 0 | 0 | 0.225 |
| 11 | 0 | 0 | 0 | 0.5 | 0 | 0 | 0.225 |
| 12 | 0 | 0 | 0 | 1.0 | 0 | 0 | 0.225 |
| 13 | 0 | 0 | 0 | 2.0 | 0 | 0 | 0.225 |
| 14 | 0 | 0 | 0 | 0 | 0.5 | 0 | 0.225 |
| 15 | 0 | 0 | 0 | 0 | 1.0 | 0 | 0.225 |
| 16 | 0 | 0 | 0 | 0 | 2.0 | 0 | 0.225 |
| 17 | 0 | 0 | 0 | 0 | 0 | 0.5 | 0.225 |
| 18 | 0 | 0 | 0 | 0 | 0 | 1.0 | 0.225 |
| 19 | 0 | 0 | 0 | 0 | 0 | 2.0 | 0.225 |

**TABLE 2. Hops Compositions**

| Example | SLS (wt %) | CAPB (wt %) | SCG (wt %) | SMCT (wt %) | LG (wt %) | POL (wt %) | Hops (wt %) |
|---|---|---|---|---|---|---|---|
| 20 | 1.0 | 0.33 | 0 | 0 | 0 | 0 | 0.225 |
| 21 | 1.0 | 0 | 0.33 | 0 | 0 | 0 | 0.225 |
| 22 | 1.0 | 0 | 0 | 0.33 | 0 | 0 | 0.225 |
| 23 | 1.0 | 0 | 0 | 0 | 0.33 | 0 | 0.225 |
| 24 | 1.0 | 0 | 0 | 0 | 0 | 0.33 | 0.225 |
| 25 | 0.33 | 1.0 | 0 | 0 | 0 | 0 | 0.225 |
| 26 | 0 | 1.0 | 0.33 | 0 | 0 | 0 | 0.225 |
| 27 | 0 | 1.0 | 0 | 0.33 | 0 | 0 | 0.225 |
| 28 | 0 | 1.0 | 0 | 0 | 0.33 | 0 | 0.225 |
| 29 | 0 | 1.0 | 0 | 0 | 0 | 0.33 | 0.225 |
| 30 | 0.33 | 0 | 1.0 | 0 | 0 | 0 | 0.225 |
| 31 | 0 | 0.33 | 1.0 | 0 | 0 | 0 | 0.225 |
| 32 | 0 | 0 | 1.0 | 0.33 | 0 | 0 | 0.225 |
| 33 | 0 | 0 | 1.0 | 0 | 0.33 | 0 | 0.225 |
| 34 | 0 | 0 | 1.0 | 0 | 0 | 0.33 | 0.225 |
| 35 | 0.33 | 0 | 0 | 1.0 | 0 | 0 | 0.225 |
| 36 | 0 | 0.33 | 0 | 1.0 | 0 | 0 | 0.225 |
| 37 | 0 | 0 | 0.33 | 1.0 | 0 | 0 | 0.225 |
| 38 | 0 | 0 | 0 | 1.0 | 0.33 | 0 | 0.225 |
| 39 | 0 | 0 | 0 | 1.0 | 0 | 0.33 | 0.225 |
| 40 | 0.33 | 0 | 0 | 0 | 1.0 | 0 | 0.225 |
| 41 | 0 | 0.33 | 0 | 0 | 1.0 | 0 | 0.225 |
| 42 | 0 | 0 | 0.33 | 0 | 1.0 | 0 | 0.225 |
| 43 | 0 | 0 | 0 | 0.33 | 1.0 | 0 | 0.225 |
| 44 | 0 | 0 | 0 | 0 | 1.0 | 0.33 | 0.225 |
| 45 | 0.33 | 0 | 0 | 0 | 0 | 1.0 | 0.225 |
| 46 | 0 | 0.33 | 0 | 0 | 0 | 1.0 | 0.225 |
| 47 | 0 | 0 | 0.33 | 0 | 0 | 1.0 | 0.225 |
| 48 | 0 | 0 | 0 | 0.33 | 0 | 1.0 | 0.225 |
| 49 | 0 | 0 | 0 | 0 | 0.33 | 1.0 | 0.225 |

**TABLE 3. Hops Beta Acids Extract Specification**

| Ingredient | Amount (wt%) |
|---|---|
| Hops Beta Acids | 45 ± 2 |
| Hops Alpha Acids | 0.4 ± 0.3 |
| Hops oils | 1.5 ± 0.5 |
| Propylene Glycol | 20 ± 15 |
| Water | < 8% |
| pH | 11 ± 0.5 |

### Solution Solubility Determination and pH Titration

A beaker containing an example composition from TABLE 1 and TABLE 2 was placed onto a magnetic stirring unit which was turned on and set to approximately 100 rpm until a gentle vortex formed. A calibrated pH probe was inserted. The surfactant/hops/NaCl solution was titrated using 1M HCl and a picture was taken when the pH reached 9, 8, 7, 6, 5, and 4.5. Each picture was graded manually according to the following scale:
- Clear: The example composition appeared crystal clear like water and yellow. The outline of the pH probe was easily observed.
- Slightly Hazy: The example composition appeared nearly completely transparent and slightly cloudy and yellowish. The outline of the pH probe was possible to discern.
- Partly Hazy: The example composition appeared mostly transparent and slightly cloudy and yellowish. The outline of the pH probe was slightly difficult to discern.
- Hazy: The example composition appeared slightly transparent but cloudy and yellowish. The outline of the pH probe was possible but difficult to discern.
- Very Hazy: The example composition appeared completely opaque and yellowish. The outline of the pH probe was not possible to discern.
- Precipitated: The example composition appeared completely opaque and cloudy white. The outline of the pH probe was not possible to discern.

In cases where the hops were not solubilized, a residue was present on the glass beaker and pH electrode that had to be removed by washing the electrode in 1% cocamidopropyl betaine in pH 10 buffer for 30 minutes thus demonstrating the phase instability in poorly solubilized compositions.

A calibration curve was used to convert the stability grades to optical density (OD) absorbance values at 600 nm using a spectrophotometer (Spectramax M2e, Molecular Devices, San Jose, CA, USA) using Ex. 6 and Ex. 9. The optical density is a background-normalized absorbance value from the spectrophotometer. As the composition becomes phase unstable, the optical density increases by an order of magnitude allowing accurate quantification of the transition between stable and unstable. Three 1.5 mL aliquots of each example were extracted during titration and placed into a micro cuvette with a 1 cm path length. OD values were determined at the pH values of 9, 8, 7, 6, 5, and 4.5 and measured within one minute following extraction ("initial OD") to determine the optical density at the nominal pH value. The average optical density at each pH value was then used to convert the graded scale to an optical density. The hops were considered effectively solubilized if the solution optical density was less than or equal to 0.136 Abs which corresponded to clear, slightly hazy, and partly hazy.

### Optical Density Results and Discussion

The OD values for the example compositions from TABLE 1 and TABLE 2 are given in TABLE 4 and TABLE 5, respectively.

**TABLE 4. Observed Hops Solubility based on Optical Density of Surfactant Solutions**

| | pH 4.5 | pH 5 | pH 6 | pH 7 | pH 8 | pH 9 |
|---|---|---|---|---|---|---|
| Ex. 1 | 2.149 | 2.149 | 2.149 | 2.149 | 2.149 | 2.046 |
| Ex. 2 | 2.046 | 2.046 | 2.046 | 2.046 | 0.350 | 0.072 * |
| Ex. 3 | 0.350 | 0.136 * | 0.136 * | 0.136 * | 0.136 * | 0.100 * |
| Ex. 4 | 0.136 * | 0.136 * | 0.136 * | 0.100 * | 0.100 * | 0.100 * |
| Ex. 5 | 0.350 | 0.350 | 0.350 | 0.136 * | 0.100 * | 0.100 * |
| Ex. 6 | 0.136 * | 0.100 * | 0.100 * | 0.100 * | 0.072 * | 0.072 * |
| Ex. 7 | 0.072 * | 0.072 * | 0.072 * | 0.072 * | 0.100 * | 0.100 * |
| Ex. 8 | 2.149 | 2.149 | 2.149 | 2.149 | 2.149 | 2.046 |
| Ex. 9 | 2.046 | 2.149 | 2.149 | 2.149 | 2.149 | 0.350 |
| Ex. 10 | 2.046 | 2.046 | 2.149 | 2.149 | 2.046 | 0.350 |
| Ex. 11 | 0.072 * | 0.072 * | 0.072 * | 0.072 * | 0.072 * | 0.072 * |
| Ex. 12 | 0.072 * | 0.072 * | 0.072 * | 0.072 * | 0.072 * | 0.072 * |
| Ex. 13 | 2.046 | 2.046 | 2.046 | 2.046 | 2.046 | 2.046 |
| Ex. 14 | 2.149 | 2.149 | 2.149 | 2.149 | 2.149 | 2.046 |
| Ex. 15 | 2.046 | 2.046 | 2.046 | 2.046 | 2.046 | 2.046 |
| Ex. 16 | 2.149 | 2.149 | 2.149 | 2.149 | 2.149 | 2.046 |
| Ex. 17 | 2.149 | 2.149 | 2.046 | 0.350 | 0.350 | 0.350 |
| Ex. 18 | 0.350 | 0.350 | 0.350 | 0.350 | 0.350 | 0.136 * |
| Ex. 19 | 0.136 * | 0.136 * | 0.136 * | 0.136 * | 0.136 * | 0.136 * |

| | | | | | | |
|---|---|---|---|---|---|---|
| * Indicates effectively solubilized solution | | | | | | |

In none of the examples in TABLE 4 does a single surfactant work to solubilize hops at every pH and surfactant concentration considered. Without a surfactant in Ex. 1, hops is not effectively solubilized at any tested pH level. In the case of SLS, a concentration of 0.5% SLS (Ex. 2) only resulted in effectively solubilized hops at a pH of about 9. While at 1% SLS (Ex. 3), hops was effectively solubilized at a pH of 5 or greater. In Ex. 4, a higher concentration of SLS allowed for effectively solubilized hops at a broader range of pH of about 4.5 or greater for 2% SLS. In the case of cocamidopropyl betaine in Ex. 5-7, the hops is more effectively solubilized at pH values of about 7 or greater for 0.5% betaine and the entire pH range tested for 1% and 2% betaine. In no instances does sodium cocoyl glutamate (Ex. 8-10) or lauryl glucoside (Ex. 14-16) effectively solubilize hops. In Ex. 11 and 12, sodium methyl cocoyl taurate effectively solubilized hops at 0.5% and 1% surfactant at all pH values. Surprisingly, a concentration of 2% SMCT (Ex. 13) did not solubilize hops at any tested values. For poloxamer (Ex. 17-19), hops was effectively solubilized at 1% poloxamer only at pH 9 and for 2% poloxamer at all pH values tested.

The solubility of hops could not be determined beforehand based on knowledge of surfactant type, concentration, and pH value. These results were unexpected based on the existing art.

**TABLE 5. Observed Hops Solubility in Surfactant Solutions**

| | pH 4.5 | pH 5 | pH 6 | pH 7 | pH 8 | pH 9 |
|---|---|---|---|---|---|---|
| Ex. 20 | 0.072 * | 0.072 * | 0.072 * | 0.072 * | 0.072 * | 0.072 * |
| Ex. 21 | 0.350 | 0.350 | 0.350 | 0.350 | 0.136 * | 0.136 * |
| Ex. 22 | 0.072 * | 0.072 * | 0.072 * | 0.072 * | 0.072 * | 0.072 * |
| Ex. 23 | 0.072 * | 0.072 * | 0.072 * | 0.072 * | 0.072 * | 0.072 * |
| Ex. 24 | 0.100 * | 0.072 * | 0.072 * | 0.072 * | 0.072 * | 0.072 * |
| Ex. 25 | 0.072 * | 0.072 * | 0.072 * | 0.072 * | 0.072 * | 0.136 * |
| Ex. 26 | 0.136 * | 0.136 * | 0.136 * | 0.136 * | 0.136 * | 0.136 * |
| Ex. 27 | 0.072 * | 0.072 * | 0.072 * | 0.072 * | 0.072 * | 0.072 * |
| Ex. 28 | 0.136 * | 0.136 * | 0.136 * | 0.100 * | 0.100 * | 0.136 * |
| Ex. 29 | 0.072 * | 0.072 * | 0.072 * | 0.072 * | 0.072 * | 0.072 * |
| Ex. 30 | 2.046 | 2.149 | 2.149 | 2.149 | 2.149 | 2.046 |
| Ex. 31 | 2.149 | 2.149 | 2.149 | 2.149 | 2.046 | 0.350 |
| Ex. 32 | 2.046 | 2.149 | 2.149 | 2.149 | 2.149 | 2.046 |
| Ex. 33 | 2.046 | 2.149 | 2.149 | 2.149 | 2.046 | 2.046 |
| Ex. 34 | 0.350 | 2.046 | 2.149 | 2.149 | 2.149 | 2.046 |
| Ex. 35 | 0.072 * | 0.072 * | 0.072 * | 0.072 * | 0.072 * | 0.072 * |
| Ex. 36 | 0.100 * | 0.100 * | 0.072 * | 0.072 * | 0.072 * | 0.072 * |
| Ex. 37 | 2.046 | 2.046 | 2.046 | 2.046 | 0.350 | 0.350 |
| Ex. 38 | 0.100 * | 0.100 * | 0.100 * | 0.072 * | 0.072 * | 0.072 * |
| Ex. 39 | 2.046 | 2.046 | 2.046 | 2.046 | 2.046 | 2.046 |
| Ex. 40 | 0.136 * | 0.136 * | 0.136 * | 0.136 * | 0.100 * | 0.100 * |
| Ex. 41 | 2.046 | 2.046 | 2.046 | 2.046 | 2.046 | 2.046 |
| Ex. 42 | 2.149 | 2.149 | 2.149 | 2.149 | 2.046 | 2.046 |
| Ex. 43 | 2.046 | 2.046 | 2.046 | 2.046 | 2.046 | 2.046 |
| Ex. 44 | 0.136 * | 0.350 | 2.046 | 2.046 | 2.046 | 2.046 |
| Ex. 45 | 0.072 * | 0.072 * | 0.072 * | 0.072 * | 0.072 * | 0.072 * |
| Ex. 46 | 0.100 * | 0.100 * | 0.100 * | 0.100 * | 0.100 * | 0.100 * |
| Ex. 47 | 0.100 * | 0.100 * | 0.100 * | 0.100 * | 0.100 * | 0.100 * |
| Ex. 48 | 0.100 * | 0.100 * | 0.100 * | 0.100 * | 0.100 * | 0.072 * |
| Ex. 49 | 0.350 | 0.136 * | 0.136 * | 0.136 * | 0.100 * | 0.100 * |

| | | | | | | |
|---|---|---|---|---|---|---|
| * Indicates effectively solubilized solution | | | | | | |

The hops solubility results in TABLE 5 show combinations of a main surfactant at 1% with a co-surfactant at 0.33%. The solubility of hops could not be determined beforehand based on knowledge of surfactant type, concentration, and pH value even when the results from TABLE 4 were additionally considered.

In the case of SLS as the main surfactant, which was not completely effective at solubilizing hops, additions of small amounts of cocamidopropyl betaine (Ex. 20), sodium methyl cocoyl taurate (Ex. 22), lauryl glucoside (Ex. 23), or poloxamer (Ex. 24) resulted in compositions that completely solubilize hops at each of the tested pH values. This was entirely unexpected considering some of these surfactants did not work to solubilize hops at all when used alone. The combination of sodium lauryl sulfate and sodium cocoyl glutamate (Ex. 21) was not effective to solubilize hops except at a pH of 8 or 9. This was surprising because 1% SLS alone (Ex. 3) was effective at a pH less than 8, but the addition of 0.33% sodium cocoyl glutamate (Ex. 21) prevented effective solubilization.

In the case of cocamidopropyl betaine as the main surfactant (Ex. 25-29), all compositions containing cosurfactants worked at all pH values investigated.

In no instance where sodium cocoyl glutamate was the main surfactant (Ex. 30-34) was the combination with a co-surfactant effective at solubilizing hops. However, surprisingly, when poloxamer was the main surfactant and sodium cocoyl glutamate was the co-surfactant (Ex. 47), the hops was effectively solubilized. This is surprising because sodium cocoyl glutamate alone was completely ineffective at solubilizing hops, and 1% poloxamer alone was only effective at a pH of about 9.

In the case of sodium methyl cocoyl taurate as the main surfactant (Ex. 35-39), compositions that additionally contained sodium lauryl sulfate, cocamidopropyl betaine, or lauryl glucoside as a co-surfactant all yielded solubilized hops at least for some of the pH values. Sodium methyl cocoyl taurate combined with sodium cocoyl glutamate (Ex. 37) or poloxamer (Ex. 39) were not effective to solubilize hops at any pH value.

In the case of lauryl glucoside as the main surfactant (Ex. 40-44), which did not work to solubilize hops alone at any pH value or concentration, combination with sodium lauryl sulfate (Ex. 40) were effective at solubilizing hops, while lauryl glucoside and poloxamer (Ex. 44) worked at pH of about 4.5. This is surprising because the combination of two surfactants that were unable to solubilize hops at any concentration were effective at solubilizing hops at a relatively low pH.

In the case of poloxamer as the main surfactant (Ex. 45-49), which worked mostly at 2% concentration, was effective at solubilizing hops in combination with any of the cosurfactants in most of the pH range investigated. In only one case where poloxamer was combined with lauryl glucoside (Ex. 49) at pH 4.5 was instability observed. Interestingly, this is the opposite of what was observed when lauryl glucoside was the main surfactant and poloxamer was the cosurfactant (Ex. 44). These results were unexpected based on the existing art.

### Evaluation of Hops Stability in Dentifrice Compositions

To explore the impact of different co-surfactants and flavors on phase stability of dentifrice formulations, a series of bases were made as described in TABLE 6. The weight percentages of individual ingredients add up to less than 100 to account for a formula "hole" that could be filled by silica, binders, sweeteners, dyes, aesthetic modifiers (e.g., mica or titanium dioxide), additional humectant/water, etc. These bases were used so that any phase instabilities manifested rapidly and visually to the naked eye.

**TABLE 6. Liquid Dentifrice Base Examples for Phase Separation Assessment**

| Ingredient | Base A | Base B | Base C | Base D |
|---|---|---|---|---|
| Sorbitol (70% Solution) | 30.000 | 48.000 | 60.000 | 41.040 |
| Glycerin | 20.000 | -- | | |
| Treated Water | 10.000 | 20.000 | | 23.200 |
| Stannous Fluoride | 0.450 | 0.450 | | 0.450 |
| Stannous Chloride (10% Silica) | 0.440 | 0.560 | | |
| Sodium Gluconate | 1.030 | 1.300 | | 1.060 |
| Sodium Citrate | 1.380 | 1.050 | | |
| Zinc Citrate | | 0.530 | | |
| Sodium Fluoride | | | 0.243 | |
| Sodium Acid Pyrophosphate | | | 3.690 | |
| Scope Mouthwash | | | 5.000 | |
| Zinc Lactate | | | | 0.250 |
| Xylitol | | | | 3.000 |
| Oxalic Acid | | 1.500 | | |
| Malonic Acid | | 1.700 | | |
| NaOH (50% Sol) | | 0.870 | 2.300 | 0.750 |
| Hole | 36.700 | 24.040 | 28.767 | 30.250 |
| Total | 100.000 | 100.000 | 100.000 | 100.000 |
| Salt Concentration (% by weight of composition including the hole) | 3.256 | 7.904 | 6.233 | 2.26 |
| Approximate Water Content (% by weight of composition including the hole) | ~31 | ~54 | ~47 | ~52 |
| Salt/Water Ratio | 0.10 | 0.15 | 0.13 | 0.04 |
| pH | 6.9 | 6.9 | 6.9 | 5.4 |

The experiment was repeated for the individual combinations of main surfactant, co-surfactant, flavor oil type, and Hops Beta Acid Extract as defined in TABLE 7A, TABLE 8A, TABLE 9A, and TABLE 10A. Flavor 1 is generally characterized as a high menthol mixed mint. Flavor 2 is generally characterized as a low menthol mixed mint. Flavor 3 is generally characterized as a mixed mint/wintergreen flavor. The flavors are blends of individual flavor components resulting in flavor compositions of different solubility and polarity characteristics that are representative of what flavors may be used generally in oral care compositions. It is not possible to account for all of the variations that an expert in the art may observe with respect to flavor composition; therefore, the differing compositions were used to indicate the utility of the phase separation measurement and liquid bases to rapidly observe phase instability. The added salt concentration was based on the concentration (wt%) of the soluble salts in the liquid bases including the hole. In the case of the Scope^{®} Mouthwash used in Base C, its salt content was not considered as it was mostly water.

The liquid bases were made by conventional methods. Humectant(s) and water were first combined until completely dissolved, then chelants and stabilizers (e.g., gluconate, citrate, pyrophosphate, oxalate, malonate) were added until completely dissolved. The zinc or fluoride salts were added, and the pH was adjusted to the range of about 5 to about 7.5. Once formed, the base was used within five days in the next step of the experiment. A quantity of liquid base was added to a 50 mL conical centrifuge tube. The main surfactant and co-surfactant were added and vortexed for 30 seconds. The flavor oil was then added and vortexed for 60 seconds. Then the Hops Beta Acid Extract was added and vortexed for 60 seconds. The liquid composition was allowed to sit for 24 hours before observing and recording phase separation characteristics.

Some compositions had no visible separation or had discontinuous droplets, while others separated into continuous layers. If a continuous layer was formed, the thickness of the layer was measured using calibrated calipers. For those examples where discontinuous droplets were observed with a layer thickness of less than 1 mm, the liquid composition was considered sufficiently stable that there would not be bulk phase separation in the presence of colloidal stabilizers like abrasive and polymer binders. For those examples where continuous layers were formed of measurable and well-defined thickness, the liquid composition was considered insufficiently stable or unstable such that there would be expected bulk phase separation even in the presence of colloidal stabilizers like abrasives and polymer binders. In general, there were no surfactant/co-surfactant blends that worked for all or that did not work for all base/flavor combinations. Each flavor/base combination had to be checked uniquely for phase stability. Similar to the optical density measurement used previously, the rapid phase separation test provided sufficient information to assess phase stability.

The example formula including Base A is described in TABLE 7A. TABLE 7B shows examples with various flavors and co-surfactant, if present, used in the formula of TABLE 7A and the physical separation results thereof.

**TABLE 7A. Example Formula Including Base A**

| Ingredient | Weight % |
|---|---|
| Base A | 63.30 |
| SLS (29% Solution) | 6.00 (1.74% neat) |
| Co-Surfactant | 0-1.00 |
| Hops Beta Acids Extract | 0.33 |
| Flavor Oil | 1.30 |
| Hole | 28.07-29.07 |

**TABLE 7B. Formulas Including Base A and Phase Separation Results and Observations**

| | Co-Surfactant | Co-Surfactant (neat wt %) | Flavor | Phase Sep Layer Thickness (mm) | Phase Sep Layer Observation |
|---|---|---|---|---|---|
| Ex. 50 | -- | -- | Flavor 1 | < 0.5* | Discontinuous droplets |
| Ex. 51 | Poloxamer | 1.00 | Flavor 1 | 6.53 | Continuous layer |
| Ex. 52 | Tween 80^{†} | 1.00 | Flavor 1 | 9.55 | Continuous layer |
| Ex. 53 | LG | 1.00 | Flavor 1 | < 1* | Discontinuous droplets |
| Ex. 54 | SMCT | 1.00 | Flavor 1 | 0* | |
| Ex. 55 | SCG | 1.00 | Flavor 1 | 0* | |
| Ex. 56 | -- | -- | Flavor 2 | 0* | |
| Ex. 57 | Poloxamer | 1.00 | Flavor 2 | 2.72 | Continuous layer |
| Ex. 58 | Tween 80 | 1.00 | Flavor 2 | 11.11 | Continuous layer |
| Ex. 59 | LG | 1.00 | Flavor 2 | < 1* | Discontinuous droplets |
| Ex. 60 | SMCT | 1.00 | Flavor 2 | 0* | |
| Ex. 61 | SCG | 1.00 | Flavor 2 | 0* | |
| Ex. 62 | -- | -- | Flavor 3 | < 0.5* | Discontinuous layer |
| Ex. 63 | Tween 80 | 1.00 | Flavor 3 | 1.22 | Continuous layer |
| Ex. 64 | SMCT | 1.00 | Flavor 3 | 0* | |
| Ex. 65 | SCG | 1.00 | Flavor 3 | 0* | |

| | | | | | |
|---|---|---|---|---|---|
| * Indicates effectively solubilized solution ^{†} Polysorbate 80 | | | | | |

In the case of compositions including Base A, SLS alone (Ex. 50, Ext. 56, Ex. 62) and SLS blended with lauryl glucoside (LG) (Ex. 53, Ex. 59), sodium methyl cocoyl taurate (SMCT) (Ex. 54, Ex. 60, Ex. 64), sodium cocoyl glutamate (SCG) (Ex. 55, Ex. 61, Ex. 65) provided effective stabilization of Hops Beta Acids Extract in the presence of all three flavors. In the case of SLS/SMCT and SLS/SCG blends, there were improvements compared to SLS alone. In the case of SLS/LG blends, there was still effective stabilization but with limited change in comparison to SLS only. Blends of SLS with poloxamer (Ex. 51, Ex. 57) and Tween 80 (Ex. 52, Ex. 58, Ex. 63) were ineffective at stabilizing the combination of Hops Beta Acids Extract and any of the flavors.

The example formula including Base B is described in TABLE 8A. TABLE 8B shows examples with various flavors and co-surfactant, if present, used in the formula of TABLE 8A and the physical separation results thereof.

**TABLE 8A. Example Formula Including Base B**

| Ingredient | Weight % |
|---|---|
| Base B | 75.96 |
| SLS (29% Solution) | 6.00 (1.74% neat) |
| Co-Surfactant | 0-1.00 |
| Hops Beta Acids Extract | 0.33 |
| Flavor Oil | 1.30 |
| Hole | 15.41-16.41 |

**TABLE 8B. Formulas Including Base B and Phase Separation Results and Observations**

| | Co-Surfactant | Co-Surfactant (neat wt %) | Flavor | Phase Sep Layer Thickness (mm) | Phase Sep Layer Observation |
|---|---|---|---|---|---|
| Ex. 66 | -- | -- | Flavor 1 | < 1* | Discontinuous droplets |
| Ex. 67 | Poloxamer | 1.00 | Flavor 1 | 3.23 | Continuous layer |
| Ex. 68 | Tween 80 | 1.00 | Flavor 1 | 0* | |
| Ex. 69 | LG | 1.00 | Flavor 1 | < 1* | Discontinuous droplets |
| Ex. 70 | SMCT | 1.00 | Flavor 1 | < 1* | Discontinuous droplets |
| Ex. 71 | SCG | 1.00 | Flavor 1 | < 1* | Discontinuous droplets |
| Ex. 72 | -- | -- | Flavor 2 | < 1* | Discontinuous droplets |
| Ex. 73 | Poloxamer | 1.00 | Flavor 2 | 3.18 | Continuous layer |
| Ex. 74 | Tween 80 | 1.00 | Flavor 2 | 0* | |
| Ex. 75 | LG | 1.00 | Flavor 2 | < 1* | Discontinuous droplets |
| Ex. 76 | SMCT | 1.00 | Flavor 2 | 0* | |
| Ex. 77 | SCG | 1.00 | Flavor 2 | < 1* | Discontinuous droplets |
| Ex. 78 | -- | -- | Flavor 3 | < 1* | Discontinuous droplets |
| Ex. 79 | Tween 80 | 1.00 | Flavor 3 | < 1* | Discontinuous droplets |
| Ex. 80 | SMCT | 1.00 | Flavor 3 | < 1* | Discontinuous droplets |
| Ex. 81 | SCG | 1.00 | Flavor 3 | < 1* | Discontinuous droplets |

| | | | | | |
|---|---|---|---|---|---|
| * Indicates effectively solubilized solution | | | | | |

In the case of compositions including Base B, SLS alone (Ex. 66, Ext. 72, Ex. 78) and SLS blended with LG (Ex. 69, Ex. 75), SMCT (Ex. 70, Ex. 76, Ex. 80), SCG (Ex. 71, Ex. 77, Ex. 81), and Tween 80 (Ex. 68, Ex. 74, Ex. 79) provided effective stabilization of Hops Beta Acids Extract in the presence of all three flavors. For Flavor 2, SLS/SMCT was an improvement versus SLS alone. Otherwise, there was still effective stabilization but with limited change in comparison to SLS only. Blends of SLS with poloxamer (Ex. 67, Ex. 73) were ineffective at stabilizing the combination of any of the flavors with Hops Beta Acids Extract. Blends of SLS/Tween 80 were effective in Base B but not in Base A.

The example formula including Base C is described in TABLE 9A. TABLE 9B shows examples with various flavors and co-surfactant, if present, used in the formula of TABLE 9A and the physical separation results thereof.

**TABLE 9A. Example Formula Including Base C**

| Ingredient | Weight % |
|---|---|
| Base C | 71.23 |
| SLS (29% Solution) | 6.00 (1.74% neat) |
| Co-Surfactant | 0-1.00 |
| Hops Beta Acids Extract | 0.33 |
| Flavor Oil | 1.30 |
| Hole | 20.14-21.14 |

**TABLE 9B. Formulas Including Base C and Phase Separation Results and Observations**

| | Co-Surfactant | Co-Surfactant (neat wt %) | Flavor | Phase Sep Layer Thickness (mm) | Phase Sep Layer Observation |
|---|---|---|---|---|---|
| Ex. 82 | -- | -- | Flavor 1 | 0* | |
| Ex. 83 | Poloxamer | 1.00 | Flavor 1 | 4.39 | Continuous layer |
| Ex. 84 | Tween 80 | 1.00 | Flavor 1 | 4.93 | Continuous layer |
| Ex. 85 | LG | 1.00 | Flavor 1 | < 1* | Discontinuous droplets |
| Ex. 86 | SMCT | 1.00 | Flavor 1 | 0* | |
| Ex. 87 | SCG | 1.00 | Flavor 1 | 0* | |
| Ex. 88 | -- | -- | Flavor 2 | 0* | |
| Ex. 89 | Poloxamer | 1.00 | Flavor 2 | 4.37 | Continuous layer |
| Ex. 90 | Tween 80 | 1.00 | Flavor 2 | 4.16 | Continuous layer |
| Ex. 91 | LG | 1.00 | Flavor 2 | < 1* | Discontinuous droplets |
| Ex. 92 | SMCT | 1.00 | Flavor 2 | 0* | |
| Ex. 93 | SCG | 1.00 | Flavor 2 | 0* | |
| Ex. 94 | -- | -- | Flavor 3 | < 1* | Discontinuous droplets |
| Ex. 95 | Tween 80 | 1.00 | Flavor 3 | 1.48 | Continuous layer |
| Ex. 96 | SMCT | 1.00 | Flavor 3 | < 1* | Discontinuous droplets |
| Ex. 97 | SCG | 1.00 | Flavor 3 | 0* | |

| | | | | | |
|---|---|---|---|---|---|
| * Indicates effectively solubilized solution | | | | | |

In the case of compositions including Base C, SLS alone (Ex. 82, Ext. 88, Ex. 94) and SLS blended with LG (Ex. 85, Ex. 91), SMCT (Ex. 86, Ex. 92, Ex. 96), and SCG (Ex. 87, Ex. 93, Ex. 97) provided effective stabilization of all three flavors in the presence of Hops Beta Acids Extract. For Flavor 3, SLS/SCG was an improvement versus SLS alone. Otherwise, there was still effective stabilization but with limited change in comparison to SLS only. Blends of SLS with poloxamer (Ex. 83, Ex. 89) and Tween 80 (Ex. 84, Ex. 90, Ex. 95) were ineffective at stabilizing any of the flavors with Hops Beta Acids Extract. Blends of SLS/Tween 80 were effective in Base B but not in Base A and Base C.

The example formula including Base D is described in TABLE 10A. TABLE 10B shows examples with various flavors and co-surfactant, if present, used in the formula of TABLE 10A and the physical separation results thereof.

**TABLE 10A. Example Formula Including Base D**

| Ingredient | Weight % |
|---|---|
| Base B | 75.96 |
| SLS (29% Solution) | 6.00 (1.74% neat) |
| Co-Surfactant | 0-1.00 |
| Hops Beta Acids Extract | 0.33 |
| Flavor Oil | 1.30 |
| 1N HCl | dropwise (pH adjust all samples to 5.4) |
| Hole | 15.41-16.41 |

**TABLD 10B. Formulas Including Base D and Phase Separation Results and Observations**

| | Co-Surfactant | Co-Surfactant (neat wt %) | Flavor | Phase Sep Layer Thickness (mm) | Phase Sep Layer Observation |
|---|---|---|---|---|---|
| Ex. 98 | -- | -- | Flavor 1 | < 1* | Discontinuous droplets |
| Ex. 99 | Poloxamer | 1.00 | Flavor 1 | 0* | |
| Ex. 100 | Betaine | 1.00 | Flavor 1 | < 1* | Discontinuous droplets |
| Ex. 101 | Tween 80 | 1.00 | Flavor 1 | 0* | |
| Ex. 102 | LG | 1.00 | Flavor 1 | < 1* | Discontinuous droplets |
| Ex. 103 | SMCT | 1.00 | Flavor 1 | < 1* | Discontinuous droplets |
| Ex. 104 | SCG | 1.00 | Flavor 1 | < 1* | Discontinuous droplets |
| Ex. 105 | -- | -- | Flavor 2 | < 1* | Discontinuous droplets |
| Ex. 106 | Poloxamer | 1.00 | Flavor 2 | 0* | |
| Ex. 107 | Betaine | 1.00 | Flavor 2 | < 1* | Discontinuous droplets |
| Ex. 108 | Tween 80 | 1.00 | Flavor 2 | 0* | |
| Ex. 109 | LG | 1.00 | Flavor 2 | < 1* | Discontinuous droplets |
| Ex. 110 | SMCT | 1.00 | Flavor 2 | < 1* | Discontinuous droplets |
| Ex. 111 | SCG | 1.00 | Flavor 2 | < 1* | Discontinuous droplets |
| Ex. 112 | -- | -- | Flavor 3 | < 1* | Discontinuous droplets |
| Ex. 113 | Poloxamer | 1.00 | Flavor 3 | 0* | |
| Ex. 114 | Betaine | 1.00 | Flavor 3 | < 1* | Discontinuous droplets |
| Ex. 115 | Tween 80 | 1.00 | Flavor 3 | 0* | |
| Ex. 116 | LG | 1.00 | Flavor 3 | 0* | |
| Ex. 117 | SMCT | 1.00 | Flavor 3 | < 1* | Discontinuous droplets |
| Ex. 118 | SCG | 1.00 | Flavor 3 | < 1* | Discontinuous droplets |

| | | | | | |
|---|---|---|---|---|---|
| * Indicates effectively solubilized solution | | | | | |

In the case of compositions including Base D, SLS alone and in combination with all the co-surfactants (Examples 98-118) were able to solubilize Hops Beta Acid Extract effectively in combination with all three flavors. The effective solubilization of Hops Beta Acid Extract for all combinations of surfactants/flavors considered in Base D stood in contrast to Bases A, B, or C where some combinations of surfactants were ineffective at solubilizing Hops Beta Acid extract for at least one of the flavors considered. The results for Base D did not help in predicting the results in the cases of Bases A, B, or C. The rapid solubilization test was effective at screening what combinations of surfactants were capable of solubilizing Hops Beta Acid extract in the presence of different flavors. The utility of the test was important because efficacy of the surfactant systems changed as the composition of the base changed. While not wishing to be bound by theory, it is believed that the increase in the added salt concentration from Base D to that of Bases A, B, or C was responsible for the change in solubilization efficacy across the different flavor and surfactant system combinations. This was either because the salt content was raised or the ratio of salt to water was increased.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

Every document cited herein, including any cross referenced or related patent or application and any patent application or patent to which this application claims priority or benefit thereof, is hereby incorporated herein by reference in its entirety unless expressly excluded or otherwise limited. The citation of any document is not an admission that it is prior art with respect to any invention disclosed or claimed herein or that it alone, or in any combination with any other reference or references, teaches, suggests, or discloses any such invention. Further, to the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. An oral care composition comprising:
hops acid; and
one or more surfactants, wherein the one or more surfactants comprise from greater than 0% to less than 2% of sodium methyl cocoyl taurate
wherein a solution comprising the hops acid and the one or more surfactants has an initial optical density of 0.136 or less determined as disclosed in the description, preferably 0.100 or less, more preferably 0.072 or less, at 600 nm in a pH range of about 4.5 to about 9.

2. The oral care composition of claim 1, wherein the one or more surfactants consists of a single surfactant.

3. The oral care composition of claims 1 or 2, wherein the one or more surfactants comprises sodium methyl cocoyl taurate as a main surfactant and a co-surfactant comprising sodium lauryl sulfate, cocamidopropyl betaine, or lauryl glucoside, wherein the main surfactant concentration is greater than the concentration of the cosurfactant.

4. The oral care composition of any one of the preceding claims, further comprising from about 0.01% to about 10%, by weight of the composition, of a metal ion source, preferably wherein the metal ion source comprises tin, zinc, or combinations thereof.

5. The oral care composition of any one of the preceding claims, wherein the hops beta acid comprises only non-hydrogenated hops beta acid.

6. The oral care composition of any one of the preceding claims, wherein the composition is a mouth rinse.
